# EUROPEAN PATENT APPLICATION

(11) **EP 2 341 146 A2**
(43) Date of publication of application: **06.07.2011**
(21) Application number: 10011056.8
(22) Date of filing: 04.07.2002
(51) Int. Cl.: C12Q 1/68, A61P 43/00

(54) **Method for determining chromatin structure**

(30) Priority: 05.07.2001 GB 0116453
(62) Divisional of application: 02743414.1
(71) Applicant: IMPERIAL COLLEGE INNOVATIONS LIMITED, London SW7 2AZ (GB)
(72) Inventor: Weinzierl, Robert, O. J, London SW7 2AZ (GB)
(74) Representative: Blance, Stephen John

(57) **Abstract**

A method of determining chromatin structure is described. The method comprises the steps of (i) fragmenting a nucleotide sequence at multiple HSs; and (ii) analysing fragments formed in step (i) from a plurality of sequences. In a preferred aspect, the present invention provides a method of determining chromatin structure in a nucleic acid sample comprising the steps of (i) treating the sample to fragment the nucleic acid therein at multiple HSs; and (ii) analysing fragments formed in step (i) from a plurality of genes.

SEQUENCE LISTING

<110>  Imperial College Innovations Limited

<120>  Method

<130>  P011106EPA

<150>  GB 0116453.2
<151>  2001-07-05

<160>  55

<170>  PatentIn version 3.0

<210>  1
<211>  24
<212>  DNA
<213>  Artificial

<220>
<223>  Oligonucleotide adapter

<400>  1
cgccagggtt ttcccagtca cgac                                              24


<210>  2
<211>  28
<212>  DNA
<213>  Artificial

<220>
<223>  Oligonucleotide adapter

<400>  2
gatcgtcgtg actgggaaaa ccctggcg                                          28


<210>  3
<211>  204
<212>  DNA
<213>  Artificial

<220>
<223>  HS Sequence

<400>  3
agcctggttt caattgtcag acttttttcc ccaaacgcaa ctccctctaa aagacgcaaa      60

gattgatatg tctggaataa aacctatcta atgggagggg gatcttgttt tttaaaagtc     120

acacaattag acacaaaacc cagacttcca catttgtggt taaatgcctc ctgtattaca     180

cagcagtgcc ctcatggaat tctc                                             204


<210>  4
<211>  204
<212>  DNA
<213>  Artificial

<220>
<223>  HS Sequence

<400>  4
aaaaaagaaa tgaactaggc aaagagagtt tggaaaaagg aacagcacat gtaaaaccca      60

ggattggcaa agacacaatc tattcaagaa attgaaatat gatctgcatt atatggtaga     120

gaagtagagg gtcaaggaaa caagatgagg ctggaatggt aagcagggtg gaaccctggc     180

aagccatttt atggatattg atat                                            204


<210>  5
<211>  204
<212>  DNA
<213>  Artificial

<220>
<223>  HS Sequence

<400>  5
tcctggaacc cgtgccttag cctttgtccc aactgtggtc aaacaattgt gcatctggtg      60

cctgtctccc ctgctggggc atcaactccc tcaacgccaa gatcatgcct ggcccagcac     120

cagtcacacc cagcagcacc ttgctggcct ctgaaccgat gtggactgaa taaatcccat     180

ccatcaccat ttccaccaca tttc                                            204


<210>  6
<211>  204
<212>  DNA
<213>  Artificial

<220>
<223>  HS Sequence

<400>  6
tatggcaaca tggatggaac tggaactgag agaaataagc caagaacaga aagttaaaca      60

ctgaatgttc tcactcatat gtggaagcta aaaaatagtt gatcttaaag acataaaaag     120

gatatgtgga tagcattgga gaatattatg ctaagtgaaa tacaccaaac acaaacaaat     180

accacatgtt ctcatatgtg gaat                                            204


<210>  7
<211>  204
<212>  DNA
<213>  Artificial

<220>
<223>  HS Sequence

<400>  7
aaaaagaaaa gaaaaaagaa aaataattat ttatgatatc caaatgtgat ttatctcagg      60

aaagcaaaag tggcacaaca tatgaaaaat caatgtaata gatcacataa tcagaatgga     120

gaaaaaaaca tgaccatctc aattgatgca gaaaaggcat ttgacaatat ccatactctt     180

tcatgataaa aactcagaaa atta                                            204

<210> 8
<211> 204
<212> DNA
<213> Artificial

<220>
<223> HS Sequence

<400> 8
gtcagtgaga caatcttcaa ggtagctcct aagtctttta gatacaacct tagtaatctc 60

ttaatagctt ctttgattct ggtattacaa aatatcccag gatcatcttg tccatttcct 120

gccccagacc tgaaatcaac catatatcca aggaaccctg gtacttttct gcgggaaaca 180

gtgttcagaa acaacaatca gagt 204


<210> 9
<211> 204
<212> DNA
<213> Artificial

<220>
<223> HS Sequence

<400> 9
gccagtgtgt gccaccatgc ctggctattt ttttgtattt ttagtagaga tggggtttcg 60

ccatcttgtc caggctggtc ttgaagtcct gagctcaagt gatccatctg tctcagcctc 120

ccaaagtgct gggattacag gtgtgagtca ctgtgcccag cctattattt tttaatattt 180

tttcttaaat tgagtcactg tttt 204


<210> 10
<211> 204
<212> DNA
<213> Artificial

<220>
<223> HS Sequence

<400> 10
gtgaaaccag tcatacccaa gctactggac tctggtcact ccatgatcct tggcaagatg 60

tctgtctatg aaccgcagtc cctctatctg taaaatgaag gatcctagac tgggactctc 120

aagctcactt gcctacaggg gccaggcagg taccttaggg aagggagaag gttggagtgt 180

tggtggagag ctgatatggt aaag 204


<210> 11
<211> 204
<212> DNA
<213> Artificial

<220>
<223> HS Sequence

<400> 11

ttactatatg gagaaaaata aagttagatt tctaccttat acaaatataa accataagta 60

atctgaaact taaatataaa aataaactgc agcgagctga gatcgcgcca ctgcactcca 120

gcctgggtga cagagcaaga ctccgtctca aaaataaaat aaaataaaaa gtcaaaacca 180

atgggaaaat gtctcttcaa actt 204

<210> 12
<211> 204
<212> DNA
<213> Artificial

<220>
<223> HS Sequence

<400> 12
tagtttcaat acttgtgagt tattacaaga aatacaactc tgctatgcaa cagaagaacc 60

gcaaatagtc attaaattac cgcacaccaa gcagcttgga gatcagatag gaaatgtttg 120

gggagagagt cctgctgaag ttccaagtct caaacaaaac ccctcaacat cagagtgcat 180

ggtgagttta aatttattgt aatt 204

<210> 13
<211> 204
<212> DNA
<213> Artificial

<220>
<223> HS Sequence

<400> 13
tttattcctt aacaaaacag aaatttggga agaatttcat ctaggtaatc aatgctcttt 60

acaagaaacc tgaaacacct gcaaaagtaa aaaaatcaca gatcaaggaa tttctgactt 120

attacaacat ggatcaacct taaggacatt atactaagtg aaataagcca gtcacaaaaa 180

gataaacact gtgtgattcc actt 204

<210> 14
<211> 204
<212> DNA
<213> Artificial

<220>
<223> HS Sequence

<400> 14
atttattggc cttggctttc ttggtgatta actttgatga caatagcatt tcacacaatg 60

tacaaacaat aaaattctgc ttcttacagg acagtgggat gatcttttgg taaacatttt 120

acttgggaac agttaggaga aattatgaat ctacaagtta atgatccaag cctcagttta 180

cctattaatt aactgaaaga attt 204

<210> 15

<211>  204
<212>  DNA
<213>  Artificial

<220>
<223>  HS Sequence

<400>  15
caggagggca gaagagtcaa taggttatta tttggccttc caggagaggg agatgtcaaa      60

gaaattcaga gtgatctgta tggctgtatc tgctctttgg gatcatttat ttctacaggc     120

tgttcacttt taaggtacca atagctgtca aaaaccctcc tttgccatca taaaatccca     180

aaactagggt tgaaatattg gcaa                                            204


<210>  16
<211>  204
<212>  DNA
<213>  Artificial

<220>
<223>  HS Sequence

<400>  16
aggcgcccgc caccacgccc gcctaatttt gtttttgtat ttttagtggt gatggggttt      60

caccgtgtta gccaggatgg tcttgatctc ctgacctcat gatctgccca catgggcctc     120

ccaaagtgct gggattacag gcatgagcca ccgtgcctgg ccctaaatat ttttaatggt     180

atgcaagcaa aatagactgt ataa                                            204


<210>  17
<211>  204
<212>  DNA
<213>  Artificial

<220>
<223>  HS Sequence

<400>  17
gagcagtaca acttaatttt cctaggcagc tttgttagca taagactctt cctaaagagc      60

cacaggtcct ccttggccat tttcctgtag tcattatata gatcaattac ctactgcccc     120

agtagtgctt cctaacaaac caccacaaac ttgctgtctt aaaaacataa tcatttgtca     180

tttatgctgc aggcttggtg ggtc                                            204


<210>  18
<211>  204
<212>  DNA
<213>  Artificial

<220>
<223>  HS Sequence

<400>  18
attgggtcat ttcccctttg gttcctcaaa gaccctcaca gcagccctgt tatgcttggc      60

tgggtttctg gctggtgaac agatgagctc tctcttccca gatcttgggg tgacgagagc 120

tatatccaca cccccaccca tagctgcctg tgcgtggctg tgatggaaac tggcttctca 180

tccaacagat ctcagtggag attg 204

<210> 19
<211> 204
<212> DNA
<213> Artificial

<220>
<223> HS Sequence

<400> 19
tgaatgcaaa aagaaattgt gatgtatgtg tttatgtgca tatgtgtgtg tgtgtattgt 60

aagtgtgtgt aatgaaatat tatccagcct taaaaaagga gatcctgcca tttgtggcaa 120

aaatgaataa acctgaagtg cattatgcca aacgaatgaa gtcagataca gaaataaaaa 180

ctacttcatg atatcactta tatg 204

<210> 20
<211> 204
<212> DNA
<213> Artificial

<220>
<223> HS Sequence

<400> 20
tggatctgga caggctgagg tggggtggag aacatgttct aaatcctaac accccacccc 60

aggtgttttt ggtgcttctt ctgtagagaa cccacagtga gatcttaaac tcctctcccc 120

acaacctcac acacactgat aaacccaaga ctacccatct ggaaaactca gtccaagagg 180

gaggatggag atgcgtgtct gggc 204

<210> 21
<211> 204
<212> DNA
<213> Artificial

<220>
<223> HS Sequence

<400> 21
agttttctcc tggggaaatc cctgttaggt tggtgagtct tacaagccat catcccacag 60

aggatttatt cctaaacatt tctacaatga aggctgagag gatctaatgt tgcagtttgg 120

taccacaact gttcccacaa ctctgtttgc tctgcctggc tggccccacc agagaactga 180

tgacagccag catctggtag gatg 204

<210> 22
<211> 204
<212> DNA

<213>  Artificial

<220>
<223>  HS Sequence

<400>  22
tgaaatgtgg tgcttggact ggcagcactg acatcaccag gaagattgtt agactctcag      60

actccaaccc cctgctaaat cagaatctga attttaacaa gatctctcag gtgatctgta     120

tgcacattaa catttaaact attgtattta aaagtagttt tttattttcc ttctctcaca     180

gactgatgta tttttaaagt agaa                                            204


<210>  23
<211>  204
<212>  DNA
<213>  Artificial

<220>
<223>  HS Sequence

<400>  23
cagtgggtct tgactcttta ttgaatttgc cagtccgtgt cttttaattg tggcttttag      60

cccatttaca tttaaggtta atattgttat ttgtgaattt gatcctgtca tcatgatgct     120

agcaggctat tttgctcatt agttgataca gtttcttcat gtgttgttga tctttatatt     180

ttggtatgtt tttacactgg ctgg                                            204


<210>  24
<211>  204
<212>  DNA
<213>  Artificial

<220>
<223>  HS Sequence

<400>  24
caggggttaa tgctgaaata caagcctcct gtccagtgat cagcctcttc ttctccaggc      60

ctgcctacat cctggtaagg aaggtgccag aattcttgcg gatctggaaa agctggcttc     120

ttccacctgc cccaaacatc tctaccaccc cctgaggaaa gcctgtcatt ttgacagctt     180

tgttcccttc acttgaggct ttta                                            204


<210>  25
<211>  204
<212>  DNA
<213>  Artificial

<220>
<223>  HS Sequence

<400>  25
tgagtgagca ttatgacctg agctccgcct cctatcagat cagctgcagc attcgattct      60

cataggagta cgaaccccaa tgtgaactgc acatgtgagg gatctaggtt acatgcactt     120

atgagaatct aatgcctgat gatctgaggt ggaaaaattt catcctgcaa ctatcctccc 180

tgccatctgt ggaaaaattg tctt 204

<210> 26
<211> 204
<212> DNA
<213> Artificial

<220>
<223> HS Sequence

<400> 26
tgtctccagc atttttatgc acttgagtgt acataagaat tctcctgagg agcttattaa 60

aaatacaggc gcctgctctc ctgctaactt cttttcagta gatctgcagt ggagcctgca 120

gtttgtattt gcaacaagta ctctgatgcc agtgtaccgt gttttgaaaa acattagttc 180

acacataatt tctcttttgt gtgt 204

<210> 27
<211> 204
<212> DNA
<213> Artificial

<220>
<223> HS Sequence

<400> 27
ttcaaatgct gaactaagaa aaatagcaat accattataa aaaataaagt tggtaaaaca 60

cctaccttaa taccacaagg ttctgaaata agagtcttcg gatctaaaac ttccacaaca 120

gcttctggaa taactgcttt cttcatgcag gacatcttga agccatagac atcatcccaa 180

aaagcaattc tatcagcatg ttta 204

<210> 28
<211> 204
<212> DNA
<213> Artificial

<220>
<223> HS Sequence

<400> 28
agttgtattc ttaaaataat cagtccatta ctatgcttta gagatgagga aaaacattga 60

ttgcgtaatt atttggttac ttgaatagct atgctttcat gatcctttta atgtgttgta 120

agtagttatt caaggaatca aaaatgaagc atcagataaa atattgctag caaacagcca 180

tttcatctct ctcacatagt ctgg 204

<210> 29
<211> 204
<212> DNA
<213> Artificial

<220>
<223>  HS Sequence

<400>  29
tctgttccat tttccacaaa gttcttttaa aacatgaaat gcccacatgt gtgtttcttc      60

tttcccttca gcagctgaat tttctgccac atatttacat gatctggtgg ccagctcatc     120

agtccattcc tctcatgtgg aaatcactgg agtgcaaaac cctctccggg tggcactgca     180

ctaattattt gtacgacatg gcta                                            204


<210>  30
<211>  204
<212>  DNA
<213>  Artificial

<220>
<223>  HS Sequence

<400>  30
ggtctaagcc ctctcctgtg cctcatttta actctaatac aataatactc attcttgttg      60

ccatgagtgg ctcttggttt cttccttttc ctaatgttta gatcttgctt tatgtgtgta     120

tatagtgatg gtctttggaa ggatacacaa ttaactggta cagcggttga ggggagttcg     180

gaaactggga gaaagtggca ccat                                            204


<210>  31
<211>  204
<212>  DNA
<213>  Artificial

<220>
<223>  HS Sequence

<400>  31
gtgagaacag actaatacag caagtaagag ggcatatttt ctttcttttt tttttgagac      60

agagtctcgc cctgtagcac agggtgaagt gcagtggtgc gatcttggct cactgcaacc     120

tctgcctccc gggttgaagt gattctcctg cctcagcctc ccgagtagct gggaccacag     180

gtgcccacca ccacgcctgg ctaa                                            204


<210>  32
<211>  204
<212>  DNA
<213>  Artificial

<220>
<223>  HS Sequence

<400>  32
tcttcagata actactctcc ttttgagaaa tacctcttgg cctactactg ggccttaata      60

gagactgaac acttaaccat gagtcactaa gttaccattt gatctgagct gctgatctga     120

acttgttgtt atatgaccca ccaagccata aagttagttg cgcacagcag gatttcatca     180

tcaaatgtca atggtaaata cgtg                                          204

<210>  33
<211>  204
<212>  DNA
<213>  Artificial

<220>
<223>  HS Sequence

<400>  33
aaaaataagt aagaacgggt gaagtttgtc tttctgtgcc tggcttattt tcactttaca      60

taatgacctc cagtttcatc cacgttgttg caaatgacag gatcttgttc tttttatgga     120

tgagtagtat tctgttgtgt atgtgtacca aattttctgt acccattcat ctattgatgg     180

atgcttagat tgcttccaac tctt                                            204

<210>  34
<211>  204
<212>  DNA
<213>  Artificial

<220>
<223>  HS Sequence

<400>  34
gggaagcctg gtctgctggt tcctcctccc ctgcagatct caggatggct aatctgggat      60

agaatcagtt tgatgaggga gcagaaaaag ccaaacccct gatctgaaat catggcagag     120

aaacctgaag ggtaacccca tttcatcttc aacgtcagaa acggagggtg gtgagagcca     180

agttgctact ggatagcatg tctt                                            204

<210>  35
<211>  204
<212>  DNA
<213>  Artificial

<220>
<223>  HS Sequence

<400>  35
atcagaaaga agacctggac atggagagga acagagcaaa gacgggctgg ggcccacagg      60

gcattgccca tctgatagtc acgatatcag cctgcaaagg gatccagaga aaaacgacca     120

ctatttctct tctgcctctc aaacctcatg caagtgtctc ttttggccaa ctctatttag     180

gatccataga gggaagggat tctg                                            204

<210>  36
<211>  204
<212>  DNA
<213>  Artificial

<220>
<223>  HS Sequence

<400> 36
aacaggtaaa gtgagaaagt gatagagaaa atgctaataa acagtgtggt atgaagatga 60

ctgtcaaagg accgctgaga tataggtaca gtgcttccct gatcctttct tttgaaggac 120

taaaggctaa agtatctgtg cctttgctcc cctgacaatc ttttctggag ctgaccattt 180

cacatttctg agttgggcct gctc 204

<210> 37
<211> 204
<212> DNA
<213> Artificial

<220>
<223> HS Sequence

<400> 37
aaaaaaaaaa aaaaaaagaa taaggttaag aacaacaaca tcctggttgg gcaccatggc 60

tcatgcctgt aatctcaaca ctgggagaat gaggcaggag gatctcttga gcccaggagt 120

ttgagaccag cctgggcagc atggtgagac cttatctcta caaaaaaatt gtgaaaatta 180

gccagatgtg gtggcctgca cctg 204

<210> 38
<211> 204
<212> DNA
<213> Artificial

<220>
<223> HS Sequence

<400> 38
tgagactaca ggaaagtgcc accatgtcca gctttttttc tttttagagg taggaatctt 60

gctttttgcc caggctggtc ttgaactatt ggcttcaagg gatcctccca ccacagcctc 120

ccaaagtgct gggatcacag gtgtgagcca ctgtgcctgg cctactttct atctttatag 180

atttacctat tctgcacatt tcat 204

<210> 39
<211> 204
<212> DNA
<213> Artificial

<220>
<223> HS Sequence

<400> 39
agctcgctga aactctttct cagagggttg ttttacacag ccttggcctc ccacccatct 60

gctgccacac ctctcagtcc tttcaagtat tatctagaga gatccaattg tcagacactg 120

agaacaaagc atgagtctca gctttattag ggatttcccc tcctataata tttcatacaa 180

agcctcattc acacccaata tgaa 204

<210> 40
<211> 204
<212> DNA
<213> Artificial

<220>
<223> HS Sequence

<400> 40
tcagaaaaaa acttccattt ataaaatagt taacaatact tttattaagt caaattcaag 60

tctaaggatt taatgtttaa aaactcgcaa aacttagtat gatctaattt ttgttttcat 120

cgttttacta ctttctacta aaatgtaaca tcacattcac taattcagaa tatcagacaa 180

aaatattaaa atttttcag aagt 204


<210> 41
<211> 204
<212> DNA
<213> Artificial

<220>
<223> HS Sequence

<400> 41
attaaacggt acattgttta gcaatacaaa cacatggtaa aactataaag aaaagcaaga 60

gtgataaaag caaaactcag ggatgacttg ggccgggtga gatcagggag gggacacagg 120

ggactccaaa ggtaacagta agttcttttt ggaaactggg taatgggtac atggaagatg 180

tttcttgtat ccttattctt taca 204


<210> 42
<211> 204
<212> DNA
<213> Artificial

<220>
<223> HS Sequence

<400> 42
tggcgggcaa agagctctgg gtaggaaaca ggagtttttt gtttggttgc ttgtttgttt 60

gctctctaga tgtcttaagt tgttgggact gctgaggaca gatccagaga atgtcataaa 120

gcttatttag ggagtagtca tcgaggtggg ccaggcactt catgtacatt atctctaatc 180

ctcacaacaa tagcagctat catt 204


<210> 43
<211> 204
<212> DNA
<213> Artificial

<220>
<223> HS Sequence

<400> 43

tgagtgaaga ttaaattttc tgtaacaggt acgatccaca taatggatta aacaaatgat 60

aagttttgct gtctttatgt aaaagaatac agaggcgggt gatccagagc tggcatagtg 120

tcctccatta acttgtcaga gacccagggt catctcagtt catcattctg ccatcccctg 180

ggtgtggccc tgggctccat ggtc 204

<210> 44
<211> 204
<212> DNA
<213> Artificial

<220>
<223> HS Sequence

<400> 44
ggccactgat ctgagcctga gggtcccggc gcctaagagg tcgggctgtc cgttccctgg 60

ggcctcctgg ccatgggcag tggcggggca cgcaggcggc gatcagaggc tgtcccgtcc 120

tctccggggg ccgcggctca tcctgccagg catctccgag gaaagtttgc tctccggaaa 180

agaagaaacc cgcgcctcct ccga 204

<210> 45
<211> 204
<212> DNA
<213> Artificial

<220>
<223> HS Sequence

<400> 45
cagggtctgc ggtcagacgt gggcaggggc catacaaaga cggtggccac aggcgtctgc 60

acatggctgt ggcttcctcc tacttcattt ccactctgca gatcttcctc tgagagcagg 120

tgtgagtccc aagaccactc tctcacatgc actcacaggc cagttctcca ccctggtgct 180

ttcaattaga aaacaaaagg tgtg 204

<210> 46
<211> 204
<212> DNA
<213> Artificial

<220>
<223> HS Sequence

<400> 46
tgtatgcaca tgccaccata cctaggtaat ttttaaagat tcttttatag agatggggtc 60

ttatgttgcc caggctcgtc tcaaactcct gggctcaagt gatcctctcg cctctgcttc 120

ccaaagtgct gggataacag gcgtgagcca ctgcacccag tcgatggcgt gtattaaaag 180

gaatgatgat atgaatactg atga 204

<210> 47

<211>  204
<212>  DNA
<213>  Artificial

<220>
<223>  HS Sequence

<400>  47
tgcttcagag gctgggatct cggtcctgga cgcccgcgct ctacgcagcg cggaagcggc      60

ggggcagtca gcctagccac cttcggaact ctccacgcct gatcccgttc cccggagccc     120

gcggccgtta ccttggtgcc tccgagccgt aacacttcct ccagggagaa cccattctcg     180

gcttcactag tattatcctc atcc                                            204


<210>  48
<211>  204
<212>  DNA
<213>  Artificial

<220>
<223>  HS Sequence

<400>  48
cgctttgggc tgcattccca agcaacccga ctccgggaag acccgggccc ggcgcgccgg      60

gggccgctac cggcctcaca ccgtccacgg gctgggcctc gatcagaagg acttgggccc     120

cccacgagcg gcgccgggga gcgggtcttc cgtacgccac atgtcccgcg ccccgccgcg     180

gggcggggat tcggcgctgg gctc                                            204


<210>  49
<211>  204
<212>  DNA
<213>  Artificial

<220>
<223>  HS Sequence

<400>  49
gaaagaaaaa aaaaaatcct tctatcaagg tggcctaaat caaaaagaat gttagctgac      60

ttgaacatca gctgctgctt cgtgaccttg attgcgttta gatcacctac attccccctg     120

cttcagaaga acccacatta tgctaatgac cctacttccc aaaatatcta accctctcac     180

atctagttaa gctagtgctg ttcc                                            204


<210>  50
<211>  204
<212>  DNA
<213>  Artificial

<220>
<223>  HS Sequence

<400>  50
cataaattac tctttaggag agttgacaaa aagttgaaac taattagctc ttgtaaggaa      60

gactgagttc tgccagtgtt gagcaatggg ttcttcttaa gatccaggca catattttaa 120

agatcaagac ataacatcat cagtatgttg aagtaatgca cagttgcatc aaaggctaaa 180

atgtggagta gagaaatgtg cctt 204

<210> 51
<211> 204
<212> DNA
<213> Artificial

<220>
<223> HS Sequence

<400> 51
agtaacacct ccccacaatt gtgacaaaca aaaatgtctc cagacattgc taaatgtccc 60

cagggggtaa aattgcccct agttgagaac cactactatg gatcctgtcc atgattatcc 120

taaatttctt tggaattaag cttttacaga gactggcaat tttcagtgtg taactgctta 180

tgcaacacag cctcattaat acaa 204

<210> 52
<211> 204
<212> DNA
<213> Artificial

<220>
<223> HS Sequence

<400> 52
aagtgctgcc ccatggcagg ggagctcact gcagtgtctg ggtctgcctg cagcatccag 60

cgtgcatggc cggcgccccc tccctcggac ctggctgccg gatcacagcc catgactcac 120

gctctgggtg cttttcactc attttcatgt tttaccctcg tgtccttcca aggaaatcac 180

atttctcaga aagaaaaagc aaac 204

<210> 53
<211> 204
<212> DNA
<213> Artificial

<220>
<223> HS Sequence

<400> 53
ctgaatttgg gatcacaaag tagaattttt aaaaagggaa atcttacctt ttgttatttg 60

cagtaaaaac agaaccgtgc atgccaacat tgcactctgt gatcttcccg ccctcagatc 120

ttcccgccct caagagactg agttctcaga ctgtactaat aaagcaatct atgtcttctt 180

ctgatttcta cctttacctc ctga 204

<210> 54
<211> 204
<212> DNA

<213>  Artificial

<220>
<223>  HS Sequence

<400>  54
   tatttacttt ttttagagac agagcctcac tctgttgtcc aggctggagt atagtactgc      60
   aatcatagct ccccgcagct tcatattcct gggctcaaga gatccttcca cctcagcctc     120
   ccacgtagtt aggactacag gcacatgcca ccatgccccg ctaagttttc gttttcttaa     180
   aaaaaaagtt tttttagaga cagg                                             204


<210>  55
<211>  204
<212>  DNA
<213>  Artificial

<220>
<223>  HS Sequence

<400>  55
cctatctttt tatttttatt atagatgggc tatcactatg ttgcccaggc tggagtgcaa      60

tggctattta caggcacaat cctgctatta atcagcatca gatctttgac ttgctctatt     120

ttcaacttgg gctgatttat ccatcctgag ataacctggt ggttctctcc ttccaggaag     180

ttaccatatt gatgccaagc ttag                                            204

## Description

### FIELD OF THE INVENTION

The present invention relates to a method and its use and products obtained therefrom.

In particular, the present invention relates to Hypersensitive Sites (HSs) and the determination of chromatin structure. Further, the present invention relates to methods for modulating (e.g. modifying) chromatin structure. Further, the present invention relates to the identification and use of chromatin modulating (e.g. modifying) agents. Further, the present invention relates to a library of HSs.

### BACKGROUND TO THE INVENTION

The large amount of DNA present in eukaryotic cells needs to be efficiently stored into a small space, the cell nucleus. This is achieved by packaging DNA molecules into chromatin, which involves looping DNA molecules around histones to create nucleosomal DNA-protein complexes. Subsequent coiling of these nucleosomal complexes into solenoid and higher order structures increases the packaging density further.

The arrangement of DNA into chromatin reduces the space taken up by DNA molecules in the nucleus, but creates an additional problem: the tight packaging of DNA prevents enzymes involved in gene expression, DNA repair and replication from accessing the genome. This situation is overcome in eukaryotic cells by exposing small selected regions of the genome to the various machineries in the form of 'Hypersensitive Sites' (HSs). For example, Nuclease Hypersensitive sites as the name implies, are genomic sites that are highly susceptible to nuclease attack under experimental conditions.

To date researchers have used HSs to obtain high-resolution 'snapshots' of the molecular microanatomy of individual HSs in particular genes.

The present invention relates to novel and useful aspects concerning HSs.

### SUMMARY ASPECTS OF THE PRESENT INVENTION

In one broad aspect, the present invention provides the analysis of HSs on a global and genome-wide scale.

In another broad aspect the present invention provides a method of determining chromatin structure of a nucleotide sequence (such as a nucleic acid sample) comprising the steps of (i) fragmenting the nucleotide sequence at multiple HSs; and (ii) analysing fragments formed in step (i) from a plurality of sequences (such as a plurality of genes).

Here, the method may be performed on a physical sample. Alternatively, the method may be performed in part *in silico.*

In another broad aspect the present invention provides 'Global Analysis of Chromatin Topology' ('**GACT**') or Hypergenomic Display ('HD').

ln a highly preferred aspect, the present invention provides methods for determining chromatin structure on a broader scale using HSs across multiple genes rather than concentrating on a small number of genes and individual HSs. The present invention also provides products obtained by the use of said method (such as a library or chromatin modulating (e.g. modifying) agents identified utilising said method).

The methods of the present invention are suitable for any organism, in particular eukaryotic organisms.

Another broad aspect of the present invention relates to the use of different restriction enzymes to introduce targeted cuts into DNA sequences present in HSs.

Another broad aspect of the present invention relates to the use of Restriction Landmark Genomic Scanning (RLGS) technique, in combination with *in situ* labeling of cleaved restriction sites, to generate two-dimensional representations of the HS sites.

Another broad aspect of the present invention relates to the use of the polymerase chain reaction in combination with an adapter oligonucleotide and a set of short arbitrary primers to generate a series of one-dimensional representations of HS sites.

Another broad aspect of the present invention relates to cross-species comparison of HS annotation data to aid interpretation of HS profiles.

Another broad aspect of the present invention relates to means of identification of individual DNA fragments in GACT profiles and HS libraries to establish a direct link between HSs and defined genomic locations.

Another broad aspect of the present invention relates to the application of GACT to map genomic topoisomerase binding sites under *in vivo* conditions.

Another broad aspect of the present invention relates to a method for preparing a library of HSs.

Other aspects of the present invention are presented in the accompanying claims and in the following description and drawings. These aspects are presented under separate section headings. However, it is to be understood that the teachings under each section are not necessarily limited to that particular section heading.

### DETAILED ASPECTS OF THE INVENTION

According to a first aspect of the present invention, there is provided a method for determining chromatin structure in a nucleic acid sample comprising the steps of (i) fragmenting the nucleotide sequence at multiple HSs; and (ii) analysing fragments formed in step (i) from a plurality of sequences.

According to a second aspect of the present invention, there is provided a method of determining chromatin structure in a nucleic acid sample comprising the steps of (i) treating the sample with a first restriction enzyme capable of fragmenting the nucleic acid at multiple HSs; (ii) incorporating detectable nucleotides into the fragments formed in step (i); (iii) isolating the nucleic acid from the sample; (iv) treating the nucleic acid from step (iii) with a second restriction enzyme; and (v) analysing the fragments from step (iv) from a plurality of genes.

In a further aspect of the present invention, there is provided a method of determining chromatin structure in a nucleic acid sample comprising the steps of (i) treating the sample with a first restriction enzyme capable of fragmenting the nucleic acid at multiple HSs; (ii) incorporating detectable nucleotides into the fragments formed in step (i); (iii) isolating the nucleic acid from the sample; (iv) treating the nucleic acid from step (iii) with a second restriction enzyme; and (v) analysing the fragments from step (iv) from a plurality of genes by applying the fragmented nucleic acids from step (iv) to gel electrophoresis incorporating an in-gel digest.

In a further aspect of the present invention, there is provided a method of determining chromatin structure in a nucleic acid sample comprising the steps of (i) treating the sample with a first restriction enzyme capable of fragmenting the nucleic acid at multiple HSs; (ii) incorporating a target nucleotide sequence into the fragments formed in step (i); (iii) isolating the nucleic acid; (iv) selectively amplifying portions of the nucleic acid from step (iii) using a first primer capable of binding to the target nucleotide sequence and a second primer capable of binding to another portion of the nucleic acid; and (v) analysing the amplified portions from step (iv).

According to a third aspect of the present invention, there is provided a method of determining a characteristic of a nucleic acid sample comprising the steps of (i) treating the nucleic acid sample to fragment the nucleic acid therein at multiple HSs; (ii) analysing fragments formed in step (i) from a plurality of genes; and (iii) using the pattern of fragments from step (ii) to determine the characteristic.

According to a fourth aspect of the present invention, there is provided a method of diagnosing a disease in subject wherein the disease is associated with an altered chromatin structure the method comprising the steps of (i) treating a nucleic acid sample from the subject to fragment the nucleic acid therein at multiple HSs; (ii) analysing fragments formed in step (i) from a plurality of genes; and (iii) using the fragment pattern to diagnose the disease.

According to a fifth aspect of the present invention, there is provided a method of monitoring the progress of a disease in a subject, the method comprising the steps of (i) treating a nucleic acid sample from the subject to fragment the nucleic acid therein at multiple HSs; (ii) analysing fragments formed in step (i) from a plurality of genes; and (iii) using the pattern of fragments from step (ii) to determine the stage of the disease.

According to a sixth aspect of the present invention, there is provided a method of testing the efficacy of a putative therapeutic agent which may be suitable for treating a disease in a subject associated with an altered chromatin structure comprising using said agent in a method according to the present invention and then determining if said agent is capable of modulating said HS.

According to a seventh aspect of the present invention, there is provided a method of identifying a chromatin modulating (e.g. modifying) agent capable of modulating (e.g. modifying) chromatin structure, the method comprising (i) treating a nucleic acid sample to fragment the nucleic acid therein at multiple HSs, wherein the sample is either pre-treated or not pre-treated with the chromatin modulating (e.g. modifying) agent; (ii) analysing the fragments formed in step (i); and (iii) comparing the fragments analysed in step (ii) to identify the chromatin modulating (e.g. modifying) agent.

According to a eighth aspect of the present invention, there is provided a method of identifying chromatin modulating (e.g. modifying) agent binding sites, the method comprising determining the chromatin structure in a nucleic acid sample with and without treatment with a chromatin modulating (e.g. modifying) agent and comparing the resulting fragment patterns to identify the location of the binding sites in the original sample.

According to a ninth aspect of the present invention, there is provided a process comprising the steps of performing a method of identifying a chromatin modulating (e.g. modifying) agent and/or a chromatin modulating (e.g. modifying) agent binding site in accordance with the present invention; identifying one or more agents capable of diagnosing and/or modulating HSs; and preparing a quantity of those one or more agents.

According to a tenth aspect of the present invention, there is provided a process comprising the steps of performing a method of identifying a chromatin modulating (e.g. modifying) agent and/or a chromatin modulating (e.g. modifying) agent binding site in accordance with the present invention; identifying one or more agents capable of diagnosing and/or modulating said HSs; and preparing a pharmaceutical composition comprising those one or more identified agents.

According to an eleventh aspect of the present invention, there is provided a process comprising the steps of performing a method of identifying a chromatin modulating (e.g. modifying) agent and/or a chromatin modulating (e.g. modifying) agent binding site in accordance with the present invention; identifying one or more agents capable of diagnosing and/or modulating said HSs; modifying those one or more identified agents; and optionally preparing a pharmaceutical composition comprising those one or more modified agents. According to a twelfth aspect of the present invention, there is provided a method of treating a disease associated with chromatin structure in a subject, the method comprising administering to the subject an effective amount of a chromatin modulating (e.g. modifying) agent capable of modulating (e.g. modifying) the chromatin structure to a non-diseased form.

According to a thirteenth aspect of the present invention, there is provided a pharmaceutical composition comprising a chromatin modulating (e.g. modifying) agent and a pharmaceutically acceptable carrier, diluent, excipient or adjuvant or any combination thereof is provided.

According to a fourteenth aspect of the present invention, there is provided a method of preventing and/or treating a disorder comprising administering a chromatin modulating (e.g. modifying) agent or a pharmaceutical comprising a chromatin modulating (e.g. modifying) agent capable of modulating (e.g. modifying) the chromatin structure to a non-diseased form wherein said agent or said pharmaceutical is capable of modulating an HS to cause a beneficial preventative and/or therapeutic effect.

According to a fifteenth aspect of the present invention, there is provided a method of preparing a HS library comprising the steps of: (i) treating a nucleic acid sample to fragment the nucleic acid therein at multiple HSs; (ii) ligating the nucleic acid fragments formed in step (i) from a plurality of sequences in to a vector; and optionally (iii) transforming the vector into a host cell to provide a library of cells.

According to a sixteenth aspect of the present invention, there is provided a library of HSs wherein each library entry comprises HSs from a plurality of genes.

### PREFERRED EMBODIMENTS

For some embodiments, preferably, the method of determining chromatin structure of a nucleotide sequence comprises fragmenting the nucleic acid by exposing the sample to at least one fragmenting means capable of fragmenting the nucleic acid at multiple HSs.

For some embodiments, preferably, the fragmenting means is an enzyme.

For some embodiments, preferably, the fragmenting means is a restriction enzyme.

For some embodiments, preferably, the method of determining chromatin structure of a nucleotide sequence comprises treating the sample with a plurality of sequence specific restriction enzymes with different target site specificities capable of fragmenting the nucleic acid at multiple HSs.

For some embodiments, preferably, the method of determining chromatin structure of a nucleotide sequence comprises analysing the fragments by incorporating detectable nucleotides into the fragments at the HSs and then detecting the incorporated nucleotides.

For some embodiments, preferably, the method of identifying chromatin modulating (e.g. modifying) agents and/or chromatin modulating (e.g. modifying) agent binding sites are used to identify a chromatin modulating (e.g. modifying) agent.

For some embodiments, preferably a chromatin modulating (e.g. modifying) is used in the preparation of a pharmaceutical composition for the treatment of an HS related disorder.

The library of HSs may comprise one or more of the 53 HS sequences set forth in SEQ ID No. 3 to SEQ ID No. 55.

### HYPERSENSITIVE SITES

Hypersensitive Sites (HSs) - such as Nuclease Hypersensitive Sites (NHSs) - are genomic sites that are highly susceptible to nuclease attack under experimental conditions - typically by approximately two orders of magnitude as compared to bulk chromatin (see Staider *et al.,* 1980; Wu, 1980). All available data suggests that HSs are mostly free of nucleosomes, but contain a number of transcription factor complexes that are bound to specific sequence motifs present in the genomic DNA.

HSs can be viewed as the gateways to the genome for the vast majority of molecules involved in regulating gene expression and many other important genomic functions, such as DNA replication, repair, recombination and insertion of retroviral genomes (reviewed in Gross and Garrard, 1988). They expose or hide gene regulatory signals and therefore constitute one of the most important epigenetic regulatory layers that are superimposed on the genome to control and direct its expression (Bonifer 2000).

HSs can be present in a number of forms - such as constitutive HSs, developmentally regulated HSs, tissue-specific HSs and cell type-specific HSs.

### CONSTITUTIVE HSs

Many 'housekeeping' genes, especially those that are expressed in a large variety of tissues and continuously during development, contain constitutive HSs that are present in most cell types. The promoter directing the expression of the human β-globin gene cluster contains several HSs that are also present in a number of non-erythroid cells (Dhar *et al.,* 1990). It is likely that the DNA present in many constitutive HSs contains primary sequence motifs that ensure HS formation in a manner that is independent of tissue-specific factors. Simmonsson *et al*. (1998) reported an unusual tetraplex DNA structure in the HS of the *c-myc* promoter, and other HSs are known to contain nuclease S1-sensitive DNA, indicating the presence of stem-loop or other unusual secondary structures (Mielke *et al.*,1996). This hypothesis is further supported by observations showing that a DNA segment containing the 'HS-2' region of the human β-globin promoter establishes a *bona fide* HS conformation, even when maintained as part of an artificial chromosome in a yeast host strain (Svetlova *et al.,* 1998). Such constitutive HSs could, in addition to regulating the expression of adjacent genes, serve as border elements to define functional chromatin domains, or could facilitate the precise folding patterns of individual chromatin fibres (Filipsky *et al*., 1990).

### DEVELOPMENTALLY REGULATED HSs

The continuous reconfiguration of chromatin architecture is an essential prerequisite for directing the changing gene expressions patterns during embryonic development and cell type-specific differentiation. Many HSs - such as developmentally regulated HSs - are created near defined subsets of genes in a tissue- and stage-specific manner (see e.g. Gross and Garrard, 1988) due to the local activity of transcription factors and chromatin remodeling machineries (reviewed in Wolffe and Hayes, 1999). The creation of HSs near genes is one of several steps in the pathway that prepares a regulatory sequence to become functionally active in chromatin. One of the best-understood model systems is the chicken lysozyme gene, where the HS configuration on its promoter has been shown to be highly dynamic. Several distinct HSs appear and disappear over different promoter elements as cells progress through haemopoetic development (Huber *et a*/., 1995; see Kontaraki *et al.,* 2000). In many cases, a direct correlation between the appearances and disappearances of HSs with known biological functions has been shown.

### PLURALITY OF GENES

In a highly preferred aspect of the present invention, a plurality of sequences, for example, a plurality of genes is used. As used herein, the term "plurality of genes" refers to at least about 3 genes and preferably is selected from the group comprising: at least about 10-15 genes; at least about 16-20 genes; at least about 21-100 genes; at least about 101-1000 genes; at least about 1001 to 5000 genes; at least about 5001 to 10000 genes; at least about 10001 to 50000 genes; and at least about 50001 to 100,000 genes; or any suitable combination of start or end points, for example, at least about 16-5000 genes.

Thus, the methods of the present invention may be used for determining chromatin structure on a broad scale using HSs across a plurality of genes.

### SAMPLE

The term "sample" as used herein includes both physical entities and representations thereof. Examples of representations include *in silico* models thereof, wherein said method is performed in part *in silico* by use of suitable analytical software tools.

In a preferred aspect, the sample is a physical entity. Here, the sample comprises nucleic acid in which chromatin topology is to be determined according to the methods of the present invention. The sample may be or may be derivable from biological material.

Preferably, the sample may be or may be derived from one of more entities selected from one or more nuclei, one or more cells, or one or more tissue samples. The nuclei, cells, or tissues may be or may be derivable from any nuclei, cells, or tissues in which chromatin is present. More preferably, the sample may be or may be derived from one of more entities selected from one or more isolated nuclei, one or more isolated cells, or one or more isolated tissue samples. The isolated nuclei, cells, or tissues may be or may be derivable from any isolated nuclei, cells, or tissues in which chromatin is present.

### FRAGMENTING MEANS

As used herein, the term "fragmenting means" refers to any entity capable of fragmenting nucleic acid.

The entity may be a chemical or physical agent such as bleomycin, bromoacetaldehyde, chloracetaldehyde, cobalt chiral complex, copper phenanthroline, diethyl pyrocarbonate, dimethyl sulfate, iron(II)-EDTA, methidiumpropyl-EDTA, neocarzinostatin, psoralen and ultraviolet light.

The entity may be an enzyme such as a sequence specific nuclease, a non-sequence specific nuclease, Bal-31, DNase I, DNase II, an endogenous nuclease, exonuclease III, lambda exonuclease, micrococcal nuclease, mung bean nuclease, *Neurospora crassa* nuclease, a restriction enzyme including type I, II and III restriction enzymes, S1 nuclease or a topoisomerases such as topoisomerase I or II.

Preferably, the entity is an enzyme. More preferably, the entity is a restriction enzyme. Even more preferably, the restriction enzyme recognises at least a 4 base pair (bp) target sequence. Most preferably, the restriction enzyme is selected from the group consisting of Dpnll, Mbol, Nlalll, SauIIIA and Tsp5091.

The methods of the present invention may involve the use of one or more such entities. For some embodiments, two different entities may be used - such as a restriction enzyme that recognises a 4 bp target sequence and a restriction enzyme that recognises a 6 bp target sequence.

### NUCLEOTIDE SEQUENCE

As used herein, the term "nucleotide sequence" is synonymous with the term "polynucleotide".

Aspects of the present invention involve the use of nucleotide sequences, which are available in databases. These nucleotide sequences may be used to express amino acid sequences that may be used as a component of the composition of the present invention. In another embodiment, the nucleotide sequences may be used to identify suitable chromatin modulating (e.g. modifying) agents for use in the composition of the present invention. In another embodiment, the nucleotide sequences may be used to verify that a chromatin modulating (e.g. modifying) agent may be used in accordance with the present invention. Aspects of the present invention also concern the use of nucleotide sequences that may comprise the fragmenting means of the present invention. Thus, in one embodiment, nucleotide sequences encoding the fragmenting means of the present invention are introduced into cells using methods well known in the art and the fragmenting means expressed therein.

The nucleotide sequence may be DNA or RNA of genomic or synthetic or recombinant origin. The nucleotide sequence may be double-stranded or single-stranded whether representing the sense or antisense strand or combinations thereof.

The nucleotide sequence may be prepared by use of recombinant DNA techniques (*e.g.* recombinant DNA).

The nucleotide sequence may be the same as the naturally occurring form, or may be derived therefrom.

### AMINO ACID SEQUENCE

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "protein".

Aspects of the present invention concern the use of amino acid sequences, which are available in databases. These amino acid sequences may comprise the chromatin modulating (e.g. modifying) agents of the present invention. In another embodiment, the amino acid sequences may be used to identify suitable chromatin modulating (e.g. modifying) agents for use in the composition of the present invention. In another embodiment, the amino acid sequences may be used to verify that a chromatin modulating (e.g. modifying) agent may be used as a chromatin modulating (e.g. modifying) agent according to the present invention.

Aspects of the present invention also concern the use of amino acid sequences that may comprise the fragmenting means of the present invention.

The amino acid sequence may be isolated from a suitable source, or it may be made synthetically or it may be prepared by use of recombinant DNA techniques.

### TREATING THE SAMPLE

In accordance with the present invention, the sample is treated to fragment the nucleic acid contained therein.

The aim of the fragmentation step is to produce a suitable number of fragments for analysis purposes. Thus, it will be appreciated that the degree to which the nucleic acid is fragmented depends, at least partly, on the nature of the starting nucleic acid sample and how many genes and HSs are contained therein. Some starting material will require more stringent fragmentation than others to produce the desired number of fragments.

The nucleic acid may be fragmented by any means apparent to one skilled in the art. Preferably, the nucleic acid is fragmented by exposing the sample to at least one fragmenting means capable of fragmenting the nucleic acid therein at multiple HSs. More preferably, the restriction enzyme leaves an overhang after fragmentation.

In a highly preferred aspect, the sample is treated with a plurality of sequence specific restriction enzymes with different target specificities capable of fragmenting the nucleic acid at multiple HSs. Preferably, the nucleic acid exposed in HSs is fragmented in a manner that produces a number of fragments that can be subsequently detected and analysed. Thus, the restriction enzyme treatment introduces a number of sequence-specific cuts into the nucleic acid at sites that are exposed to the nuclease because they are present in an HS configuration.

The present invention represents methodology that allows for the entire genome or portions thereof to be conveniently analysed. Using the present invention, a large number of data points may be derived from a single experiment. This renders the present invention ideally suited to bioinformatic processing and analysis of genetic information. The nature of the invention also makes it particularly suitable for automation, including robotic systems that further add to the ability of the invention to conveniently and efficiently produce substantial amounts of useful information from nucleic acid samples. One application where this efficiency is particularly useful is in high throughput drug screening.

In a preferred aspect of the present invention, if the sample comprises one or more nuclei, one or more cells, or one or more tissues then the sample may be treated to enable the fragmenting means to come into contact with the chromatin therein.

In another preferred aspect of the present invention, if the sample comprises one or more isolated nuclei, one or more isolated cells, or one or more isolated tissues then the sample may be treated to enable the fragmenting means to come into contact with the chromatin therein.

In one embodiment of the present invention, the cells may be permeabilised or otherwise treated to enable the fragmenting means to pass through the cell membrane and come into contact with the nucleic acid inside the cell.

For example, the sample may be temporarily permeabilised by lysolecithin treatment either *in vitro* or *in vivo* and then incubated in a buffer containing a specific restriction enzyme. The restriction enzyme enters the cell and cleaves nucleic acid target sites that are exposed in HSs. Other target sites that are present in conventional chromatin configurations are not affected under these circumstances. After allowing time for cleavage, the cells may even be permeabilised again, if required.

### ANALYSING FRAGMENTS

The method of the present invention utilises an analytical step - wherein the prepared fragments are analysed. As used herein, the term "analysed" refers to the visualisation and/or identification and/or detection and /or mapping and/or sequencing of fragments. It is to be noted that the analysis need not necessarily be by the naked eye. By way of example, the analysis may be conducted by computer aided technology - such as image analysis.

The fragments may be analysed by separating them on the basis of distinct characteristics such as size and charge. Preferably, the fragments are analysed using gel electrophoresis. In this regard, the fragments may be subjected to gel electrophoresis with various parameters, as required, to adequately analyse the fragments. These parameters include: the physical dimensions of the gel *i.e*. larger gels provide improved resolution and thus can reveal larger numbers of fragments; and the nature and concentration of the gel matrices and buffers *i.e*, short DNA fragments will be better resolved on high percentage gels, whereas longer fragments may need to be resolved in low percentage gels. It will be appreciated that the number and nature of the fragments in the mixture will affect the parameters chosen for the electrophoresis and that the choice of the most appropriate electrophoresis system is well within the knowledge of a skilled person.

The fragments may be analysed by incorporating detectable nucleotides into the fragments at the HSs and then detecting the incorporated nucleotides. In one particular form, the detectable nucleotides are tagged nucleotides capable of being incorporated into the fragments using an enzyme such as DNA polymerase. The tag may be varied and may include radioactive and non-radioactive labels - such as fluorescent labels.

The fragments may be analysed by DNA sequencing. Thus, in one embodiment of the present invention, a library of. The sequenced inserts may be searched against a database - such as human genome database - and the precise genomic location of the HS determined.

In some instances the composition of fragments obtained after fragmentation at the HSs may require further fragmentation to form a suitable number and pattern of fragments for analysis. This additional fragmentation step may be carried out by any one of a number of methods such as using an additional fragmentation step. When the fragments are to be analysed using gel electrophoresis, the additional fragmentation step may be carried out as an in-gel digest using a suitable fragmenting means adapted to cut the fragments during the electrophoresis. The suitable fragmenting means may be varied and preferably comprises a restriction enzyme with at least a 4 bp cutting recognition site.

The choice of fragmenting means used determines the number of HSs cleaved and thus the numbers of fragments produced. For example, If the fragmenting means is a restriction enzyme with a 4 bp recognition sequence then this will result in a larger number of HSs being cleaved and thus has a direct effect on the number of fragments. In addition to utilizing a simple scoring method (*e.g*. fragment present or absent) for analysing the results of the methods of the present invention, some fragments may display an intermediate signal level, indicating that the corresponding HSs are differentially accessible in different cells. This may reflect a heterogeneity in the cell population used for the assay (*e.g*. presence of different cell types), or otherwise indicate the presence of HSs that are undergoing transitions in their structure due to physiological processes. The methods of the present invention are therefore capable of providing a large number of fragments that reflect quantitatively the states of thousands of HSs in a single experiment

In one embodiment of the present invention, the sample is fragmented by temporarily permeabilising a sample by lysolecithin treatment and then incubating the sample in a buffer containing a restriction enzyme. The enzyme enters the cell and cleaves nucleic acid target sites that are exposed in HSs. After allowing time for cleavage, the cells are permeabilised again and incubated with a DNA polymerase (such as the Klenow fragment or 'Sequenase') and at least one labeled dNTP. The label may be any label that allows subsequent analysis - such as a radioactive label or a fluorescent label. This leads to the selective incorporation of the label into the nucleic acid at the sites that have been cleaved by the restriction enzyme. After allowing time for incorporation, the cells are lysed and the nucleic acid purified by methods well known in the art. The nucleic acid may then be cleaved with a second restriction enzyme - such as a restriction enzyme with a 6 bp recognition sequence - to reduce the average size of the fragments. The fragment mixture is subjected to an in-gel digest with a 4 bp cutting restriction enzyme to produce a two-dimensional gel image. The labeled fragments may be subsequently analysed by a number of methods well known in the art - such as phospho-imaging and/or autoradiography. Each detectable fragment or spot on the two-dimensional gel image corresponds to a cleaved HS, and therefore the entire gel image represents a global map of the chromatin topology present in the original cell sample. This generates a distinct pattern of fragments that reflects the presence of HSs on a genome-wide level in a quantitative manner.

### AMPLIFICATION

Fragments generated in the methods of the present invention may also be analysed after being amplified. The amplification may be carried out using any one of a number of techniques apparent to those skilled in the art such as the polymerase chain reaction.

The primers (or 'hypertags') used to amplify the fragments may be capable of detection to assist in the analysis of the fragments. Thus, at least one of the primers may incorporate a tag to facilitate its detection. The tag may be varied and includes fluorescent labels such as those that would render the fragments capable of detection using a fluorescent DNA analysis system. When primers incorporate a tag it may be the same tag or each primer may incorporate different tags that are distinguishable when analysed. When the primers include a tag the amplified nucleotides may be analysed by separating them on the basis of size, such as gel electrophoresis, and then detecting the tag.

The primer (or adapter primer) may have a sequence specifically chosen to match a compatible "sticky end" of a fragment generated in the fragmentation process - such as BamHl adapter primer. Alternatively, the primers may have an arbitrary sequence selected to control the number of fragments amplified.

When one of the primers has an arbitrary sequence, it may have at least 5-8 nucleotides. In this regard, longer primers will hybridise with higher stringency and as the temperature of the hybridisation phase is increased so is the stringency of the hybridisation. Thus, it will be appreciated that by adjusting the primers length and sequence and the hybridisation temperature, the number of fragments amplified can be manipulated, as required. Unwanted artifacts, such as homodimers or primers that have not hybridised may be removed prior to amplification using standard techniques such as size exclusion chromatography.

The target nucleotide may be incorporated into the fragments using any one or more of a number of enzymes adapted to perform such a task such as DNA ligase.

Thus, in one embodiment of the present invention, an 'adapter' oligonucleotide is added that contains a single-stranded region which is complementary to the 'sticky' end produced by a restriction enzyme. The adapter oligonucleotide is covalently linked to the cleaved sequence in the genomic DNA with DNA ligase. This step results in the specific addition of a short stretch of known sequence to each DNA sequence that is exposed as a HS. Genomic DNA is subsequently extracted and purified using methods well known to a person skilled in the art. Any unincorporated adapter oligonucleotides (or homodimers formed during the ligation reaction) may be removed using a method such as size-exclusion chromatography. The DNA fragment to which the adapter oligonucleotide has specifically ligated is selectively analysed by PCR using a particular DNA primer combination. For example, one of the primers may be complementary to the sequence of the adapter oligonucleotide and may be fluorescently labeled. The other primer may be a short oligonucleotide of arbitrary sequence. When the PCR reaction is carried out under 'low stringency' conditions (*i.e*. low hybridising temperatures, typically ranging between 45-55 °C) a subset of the genomic DNA will be specifically amplified and the resulting DNA fragments will contain an incorporated fluorescent label.

To improve the efficiency of the procedure, the different primer combinations may be labeled with different fluorescent labels so that the resulting PCR products can be analysed within a single capillary of a fluorescent DNA analysis system.

All of these steps may be implemented using robotic systems and may be automated in their entirety and used in high-throughput screening.

Different sizes of primers - such as hypertags and adapter primers - may allow the analysis of a variety of HSs - such as the analysis of a variety of HSs in a single electrophoresis lane.

A collection of HS-specific primers - such as hypertag and adapter primers - may be used to probe the state of a plurality of HSs. Preferably, a collection of HS-specific primers - such as hypertag and adapter primers - are used to probe the state of a plurality the most variable and diagnostically useful HSs.

The collection of HS-specific primers may be used to probe the state of a plurality of HSs in a single experiment.

### HS LIBRARIES

As used herein, the term "HS libraries" refers to a physical library of HSs - such as a library of HS fragments that may be ligated in to a vector - and/or a collection of HSs - such as a collection of HSs comprising a database of fragment fingerprints indicative of chromatin structure generated using the methods of the present invention - and/or a collection of HS specific primers - such as hypertag and adapter primers - that can be used to probe the state of a plurality of HSs.

The methods of the present invention may be used to prepare HS libraries.

The physical library of HS fragments may be prepared by treating a nucleic acid sample to fragment the nucleic acid therein at multiple HSs, followed by ligation of the nucleic acid fragments from a plurality of sequences in to a vector - such as a cloning vector - using methods well know in the art. To maximise cleavage in the majority of sites, the restriction enzyme chosen will preferably cut a 4 bp target sequence and produce a 'sticky end' suitable for cloning - such as Mbol. The cleaved genomic DNA is ligated to a vector - such as a BamHi cleaved and phosphatased vector. This step covalently joins the DNA sequences adjacent to the Mbol cleavage site to the plasmid DNA.

Since the other end of the genomic fragment may be tens of kilobases away and may also be randomly sheared during the genomic DNA extraction step, it may not be suitable for specifically joining the other BamHl site in a linearised plasmid. Thus, the ligation mixture may be cut to completion with a restriction enzyme such as EcoRl. This enzyme cuts at a defined site within the polylinker of the plasmid vector and also cuts every target site present in the genomic DNA. Since the DNA fragments adjacent to the Mbol sequence are already ligated to the plasmid vector, this step creates the condition for specifically joining the other end of the construct through intramolecular ligation. Due to the random orientation of the plasmid vector relative to the Mbol fragment during the first ligation approximately 50% of the clones may be lost at this stage, but the other 50% of ligation products will contain specifically cloned Mbol-EcoRl genomic fragments.

Optionally, the method for preparing the library of HS fragments comprises the additional step of transforming the vector to provide a library of cells using methods well known in the art and results in the creation of a physical library of genomic DNA fragments derived from a large variety of HSs.

The fragments may be selectively amplified prior to preparing the library.

The present invention also provides a collection of HSs wherein each entry comprises HSs from a plurality of genes. The collection of HSs are preferably provided in digital or electronic form to enable them to be efficiently and conveniently screened using computers. Thus, the present invention also provides a collection of HSs in the form of an electronic or digital database.

In one embodiment of the present invention, the collection of HSs produced according to the present invention may be cross-referenced with characteristics of the cells from which the nucleic acid samples were originally derived. The collection of HSs of the present invention may then be used as a reference database for determining cellular characteristics using a fingerprint obtained from a nucleic acid sample.

The HS library may also refer to a collection of HS-specific primers - such as hypertag and adapter primers - used to probe the state of a plurality of HSs. Preferably, a collection of HS-specific primers - such as hypertag and adapter primers - are used to probe the state of a plurality the most variable and diagnostically useful HSs. The collection of HS-specific primers may be used to probe the state of a plurality of HSs in a single experiment.

### CELLULAR CHARACTERISTICS

According to the present invention, the pattern of fragments obtained in the methods described herein may be used to determine the characteristics of a nucleic acid sample by comparing the pattern with a HS library - such as a collection of HSs which contain HS fragment patterns indicative of various characteristics. Cellular characteristics that correlate with chromatin structure may include; development stage, presence or absence of a disease and the state of a disease, type or form of disease *e.g*. form of cancer, age and cell type such as progenitor cell type.

### APPLICATIONS

As indicated above, HSs are an important regulatory access point for external agents to act upon the genome. Thus, it will be appreciated that the methods of the present invention have many applications in biotechnology and medicine. The methods of the present invention are broadly applicable to all eukaryotic genomes and allow for the profiling of one or more cells, one or more nuclei or one or more tissue samples based on their chromatin structure. The methods of the present invention are also broadly applicable to all eukaryotic genomes and allow for the profiling of one or more isolated cells, one or more isolated nuclei or one or more isolated tissue samples based on their chromatin structure. In particular, chromatin from certain diseased cells, nuclei, or tissues has an altered chromatin structure relative to the chromatin from otherwise healthy cells.

Much of the current experimental work in biotechnology relates to the identification of various human gene regulatory sequences. By way of example, enhancers contain clusters of transcription factor binding sites, and can stimulate the activity of adjacent genes substantially. Enhancers also play an important role in directing tissue-specific gene expression programmes. Other gene regulatory regions, such as silencers, are involved in switching off the expression of nearby genes. Research carried out over the last two decades has established a strong link between the locations of enhancers and silencers with HSs (reviewed in Gross and Garrard, 1988; Bonifer, 2000). The ability to determine chromatin structure according to the methods of the present invention in various eukaryotic genome sequences (especially the human genome) may have the potential to identify constitutive and tissue-specific enhancer and silencer sequences. These identified enhancer and silencer sequences may be suitable for numerous applications in gene therapy. Constitutive enhancers that are active in a wide spectrum of cell types can be used to promote the expression of a target gene in any cell type. Tissue-specific enhancers provide an enhanced level of specificity and can be used to promote the expression of target genes in a particular tissue, or in a restricted range of cell types. On the other hand, silencers can be used to switch of the expression of unwanted genes (e.g. oncogenes) or to silence the expression of parasitic genomes (e.g. during viral infections). Examples to illustrate this approach can be found in Smith *et al* (2000) and Phylactides *et al.* (2002). These researchers mapped a small number of HSs (using conventional experimental means) with the goal of identifying regulatory elements and enhancer regions that confer cell type-specific and correct temporal control of the expression of the cystic fibrosis transmembrane conductance regulator (CFTR) gene. The identified enhancers can be used to direct the tissue- and stage-specific expression of a synthetic CFTR gene in future gene therapeutic applications. In contrast, Harland *et al.* (2002) specifically set out to identify an enhancer that confers ubiquitous expression to adjacent genes. They successfully analysed the promoter of the universally expressed transcription factor TATA-binding protein (TBP) for the presence of a DNAase I hypersensitive site indicative of the location of such enhancers. The experimental approaches using other methods, as illustrated by Smith *et al.* (2000), Phylactides *et al.* (2002) and Harland *et al*. (2002), are capable of identifying only a small number of HSs, are laborious and can only yield information concerning small regions of the chromatin structure of the genome. In contrast, the methods described herein, may be applied to large regions of genomes.

The determination of chromatin structure according to the present invention also has numerous implications for transcription factor-based therapeutic applications. Ma *et al*, (2002) identified two transcription factors binding to DNA sequences present in a HS near the platelet-derived growth factor (PDGF)-A gene (implicated in tumorigenesis, metastasis and tumour progression). These transcription factors are repressors and are capable of diminishing the transcription of the PDFG-A gene. The identified transcription factors may play an important role in dampening the expression of this oncogenic growth factor. The methods described herein may be the starting point of larger screens to identify transcription factors capable of interacting with them.

Liu *et al*. (2001) and Zhang et al. (2000) used conventional experimental methods to map the locations of a small number of HSs near the vascular endothelial growth factor A (VEGF-A) erythropoietin genes. The aim of their work was to identify genomic regions present in nuclease hypersensitive configuration that would be suitable for binding of transcription factors containing artificial DNA-binding domains capable of binding to the exposed DNA sequences. The methods described herein may improve this process significantly.

According to the methods of the present invention, a disease associated with an altered chromatin structure may be diagnosed in a subject. The most convenient way to diagnose the disease is to compare the fragment pattern with a HS library - such as a collection of HSs comprising a database of fragment fingerprints indicative of the disease. Preferably, the disease associated with altered chromatin structure is selected from the group consisting of: cancer, chronic diseases, aging and genetic diseases.

Furthermore, when particular diseases have characteristic chromatin structures, the methods of the present invention may be used to diagnose the particular form or type of a disease. For example, the particular form of cancer afflicting a subject may be determined by determining the chromatin structure in the subject's diseased cells and comparing them with chromatin structures indicative of particular forms of cancer. As indicated previously, for convenience the comparison may best be carried out using a HS library - such as a collection of HSs comprising a computer database of fragment patterns generated using the methods of the present invention. The detailed and accurate diagnosis of disease forms such as cancer facilitates the correct choice of therapeutic treatment for the disease and thus increases the chances of successfully treating the disease.

### DISEASE PROGRESSION

Disease progression may be associated with changes in chromatin structure in affected cells. Thus, in addition to diagnosing diseases, the present invention may also be used to monitor the progress of a disease in a subject. For example, the progression of a particular type of cancer afflicting a subject may be determined by determining the chromatin structure in the subject's diseased cells and comparing them with chromatin structures indicative of the progression of a particular type of cancer. As indicated previously, for convenience the comparison may best be carried out using a library of HSs - such as a collection of HSs in a computer database of fragment patterns generated using the methods of the present invention.

### CELLULAR DEVELOPMENT

Chromatin structure may also be an indicator of cellular development. In this regard, cells at different stages of development have unique chromatin structures and hence different HSs. Thus, the present invention may be used to monitor cell development in a cell population.

Multiple samples may be taken to enable cell development, disease progression or efficacy to be determined. In such situations the determination is relative, based on differences in HSs between samples. However, it will be appreciated that the same result can be achieved by comparing the fragment pattern from a test sample with a library of HSs - such as a collection of HSs in a database of fragment fingerprints indicative of cellular development.

### CHROMATIN MODIFICATION

The methods of the present invention facilitate the generation of a substantial amount of information on chromatin structure and more particularly the location, sequence and role of HSs within chromatin. Once the sequence and role of particular HSs has been determined using the present invention, they may be modified to alter the expression of genetic information from chromatin.

The nucleic acid sequences in chromatin may be modified using standard techniques, such as site directed mutagenesis, to either include or remove one or more HSs. These modifications to the nucleic acid sequence will in turn affect the HSs and chromatin structure and the expression of genetic information therein.

As an alternative to modulating (e.g. modifying) chromatin structure by altering the nucleic acid sequence, chromatin may be modified using agents that act in a more general fashion to cut and reshape chromatin (and hence the HSs) without necessarily altering individual nucleotides. In this regard, the present invention also enables the identification and characterisation of such chromatin modulating (e.g. modifying) agents. More particularly, the ability of the methods of the invention to provide information on chromatin structure facilitates the screening of potential new chromatin modulating (e.g. modifying) agents and enables known agents to be better characterised.

Thus, the present invention may also be used to identify one or more agents capable of modulating (e.g. modifying) chromatin structure. Preferably, the agents act directly on the chromatin in the sample to modify its structure by binding to the chromatin and affecting one or more HSs. Alternatively, the agent may affect the formation or expression of HSs in the chromatin.

As well as identifying chromatin modulating (e.g. modifying) agents, the present invention also enables the identification of binding sites for chromatin modulating (e.g. modifying) agents. In this regard, the present invention may be used for identifying chromatin modulating (e.g. modifying) agent binding sites. Preferably, the chromatin modulating (e.g. modifying) agent is selected from the group comprising topoisomerases.

### CHROMATIN STRUCTURE

Chromatin structure reflected by the HSs therein affects the expression of the encoded nucleic acid and in turn the functioning of the cell. The methods of the present invention facilitate the control of cellular functions by modulating (e.g. modifying) chromatin structure and thus the expression of the genetic information. Thus, the present invention may also be used to treat a nucleic acid sample to control its expression.

The pre-determined form may be any chromatin structure that has an effect on the functioning of a cell containing the chromatin. The predetermined form may be a structure that predisposes a cell to differentiate in a particular way. In this regard, the invention may be used to prepare customised cell populations from progenitor cells. For example, once the chromatin structure that predisposes a cell to differentiate in a particular way has been determined, progenitor cells may be treated to modify their chromatin structure as necessary to predispose cells to differentiate into particular cell types. The, control of differentiation by modulating (e.g. modifying) chromatin structure enables the production of any desired cell population or the production of a uniform progenitor population with the ability to differentiate into a given cell type or types. This form of the invention may have particular application in embryonic and somatic stem cell therapy as it enables monitoring of the uniformity of the differentiation state of cell populations for administration to subjects to maximise the effectiveness of the therapy. By monitoring chromatin states it may also be possible to devise protocols capable of guiding undifferentiated embryonic stem cells into specified differentiation pathways in a stepwise and controlled manner.

The predetermined form may also be a chromatin structure that is capable of expressing a nucleic acid sequence contained therein in a preferred fashion relative to unmodified chromatin. This form of the invention may be particularly useful where the expression of the gene of interest is maximised for therapeutic purposes, such as in gene therapy.

As an extension to this form of the invention, the present invention is particularly useful in the design and production of gene constructs, including those used for gene therapy applications and in transgenics. In this regard, in addition to other regulatory and control sequences in the construct, the present invention enables a skilled person to design a construct adapted for optimal presentation in the chromatin to which it is inserted. Constitutive HSs may serve as border elements that define functional chromatin domains or may facilitate the precise folding patterns of individual chromatin fibres. Thus, constructs designed for optimal presentation in the chromatin will define one or more HSs that will ensure correct chromatin structure and in turn enable the most efficient expression of the inserted nucleic acid.

For therapeutic applications the predetermined form may also be a chromatin structure that corresponds to a non-disease phenotype. In this regard, chromatin modulating (e.g. modifying) agents may also be used to treat diseases related to chromatin structure. For example, cancer may be treated by administering a chromatin modulating (e.g. modifying) agent that modifies the chromatin in a cancer cell to prevent it from uncontrolled division. The particular agents used to modify the chromatin for therapeutic purposes will depend on the nature of the chromatin changes required. However, once the chromatin structure corresponding to a diseased phenotype has been identified using the methods of the present invention, agents may be selected that are adapted to alter particular aspects of chromatin structure for therapeutic benefit.

### AGENTS

The agents identified using the method of the present invention may be used for diagnostic purposes (i.e. a diagnostic agent) and/or for therapeutic purposes (i.e. a therapeutic agent).

The agent may be an organic compound or other chemical. The agent may be a compound, which is obtainable from or produced by any suitable source, whether natural or artificial. The agent may be an amino acid molecule, a polypeptide, or a chemical derivative thereof, or a combination thereof. The agent may even be a polynucleotide molecule - which may be a sense or an anti-sense molecule. The agent may even be an antibody.

### THERAPEUTIC AGENTS

The present invention may be used to test therapeutic agents that effect chromatin structure in a subject. For example, the chromatin structure in a subject administered with a therapeutic agent may be determined by determining the chromatin structure in the subject's cells and comparing them with chromatin structures from a subject not being tested with the therapeutic agent. As mentioned previously, for convenience the comparison may best be carried out using a HS library such as a computer database of fragment patterns generated using the methods of the present invention.

Furthermore, the present invention may be used to monitor the efficacy of a therapeutic agent capable of treating a disease in subject.

### CHROMATIN MODULATING AGENT

The methods of the present invention may be used to identify one or more agents that modulate (e.g. modify) chromatin, compositions for use in medicine comprising at least one chromatin modulating (e.g. modifying) agent of the present invention and methods of using chromatin modufating (e.g. modifying) agents of the present invention in the preparation of a medicament for the treatment of diseases.

As used herein, the term "chromatin modulating agent" may refer to a single entity or a combination of entities.

The chromatin modulating agent may be an organic compound or other chemical. The chromatin modulating agent may be a compound, which is obtainable from or produced by any suitable source, whether natural or artificial. The chromatin modulating agent may be an amino acid molecule, a polypeptide, or a chemical derivative thereof, or a combination thereof. The chromatin modulating agent may even be a polynucleotide molecule - which may be a sense or an anti-sense molecule. The chromatin modulating agent may even be an antibody.

The chromatin modulating agent may be designed or obtained from a library of compounds, which may comprise peptides, as well as other compounds, such as small organic molecules.

By way of example, the chromatin modulating (e.g. modifying) agent may be a natural substance, a biological macromolecule, or an extract made from biological materials such as bacteria, fungi, or animal (particularly mammalian) cells or tissues, an organic or an inorganic molecule, a synthetic agent, a semi-synthetic agent, a structural or functional mimetic, a peptide, a peptidomimetics, a derivatised agent, a peptide cleaved from a whole protein, a peptide synthesised synthetically (such as, by way of example, either using a peptide synthesizer or by recombinant techniques) or combinations thereof, a recombinant agent, an antibody, a natural or a non-natural agent, a fusion protein or equivalent thereof and mutants, derivatives or combinations thereof.

The chromatin modulating (e.g. modifying) agent may be an organic compound. Typically the organic compounds may comprise two or more hydrocarbyl groups. Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. The chromatin modulating (e.g. modifying) agent may comprise at least one cyclic group. The cyclic group may be a polycyclic group, such as a non-fused polycyclic group. The chromatin modulating (e.g. modifying) agent may comprise at least one of said cyclic groups linked to another hydrocarbyl group.

The chromatin modulating (e.g. modifying) agent may contain halo groups. Here, "halo" means halogen compounds eg. halides and includes fluoro, chloro, bromo or iodo groups.

The chromatin modulating (e.g. modifying) agent may contain one or more of alkyl, alkoxy, alkenyl, alkylene and alkenylene groups - which may be unbranched- or branched-chain.

The chromatin modulating (e.g. modifying) agent may be in the form of a pharmaceutically acceptable salt - such as an acid addition salt or a base salt - or a solvate thereof, including a hydrate thereof. For a review on suitable salts see Berge et al, J. Pharm. Sci., 1977, 66, 1 - 19.

The chromatin modulating (e.g. modifying) agent of the present invention may be capable of displaying other therapeutic properties.

The chromatin modulating (e.g. modifying) agent may be used in combination with one or more other pharmaceutically active agents.

if combinations of active agents are administered, then they may be administered simultaneously, separately or sequentially.

### EXPRESSION OF FRAGMENTING MEANS

In a preferred aspect of the present invention, the sample used in the methods of the present invention may be capable of expressing the fragmenting means. In this regard, the sample may be capable of expressing a nuclease or other fragmenting means capable of fragmenting the nucleic acid at multiple HSs. The nuclease or other fragmenting means may be endogenous or exogenous and preferably are encoded by an expression vector inserted into the cell or tissue using various methods well known to a person skilled in the art.

### STEREO AND GEOMETRIC ISOMERS

The chromatin modulating (e.g. modifying) agents may exist as stereoisomers and/or geometric isomers - *e.g*. they may possess one or more asymmetric and/or geometric centres and so may exist in two or more stereoisomeric and/or geometric forms. The present invention contemplates the use of the entire individual stereoisomers and geometric isomers of those chromatin modulating (e.g. modifying) agents, and mixtures thereof. The terms used in the claims encompass these forms, provided said forms retain the appropriate functional activity (though not necessarily to the same degree).

### PHARMACEUTICAL SALT

The chromatin modulating (e.g. modifying) agent may be administered in the form of a pharmaceutically acceptable salt.

Pharmaceutically-acceptable salts are well known to those skilled in the art, and for example include those mentioned by Berge et al, in J. Pharm. Sci., 66, 1-19 (1977). Suitable acid addition salts are formed from acids which form non-toxic salts and include the hydrochloride, hydrobromide, hydroiodide, nitrate, sulphate, bisulphate, phosphate, hydrogenphosphate, acetate, trifluoroacetate, gluconate, lactate, salicylate, citrate, tartrate, ascorbate, succinate, maleate, fumarate, gluconate, formate, benzoate, methanesulphonate, ethanesulphonate, benzenesulphonate and p-toluenesulphonate salts.

When one or more acidic moieties are present, suitable pharmaceutically acceptable base addition salts can be formed from bases which form non-toxic salts and include the aluminium, calcium, lithium, magnesium, potassium, sodium, zinc, and pharmaceutically-active amines such as diethanolamine, salts.

A pharmaceutically acceptable salt of a chromatin modulating (e.g. modifying) agent may be readily prepared by mixing together solutions of a chromatin modulating (e.g. modifying) agent and the desired acid or base, as appropriate. The salt may precipitate from solution and be collected by filtration or may be recovered by evaporation of the solvent.

A chromatin modulating (e.g. modifying) agent may exist in polymorphic form.

A chromatin modulating (e.g. modifying) agent may contain one or more asymmetric carbon atoms and therefore exist in two or more stereoisomeric forms. Where a chromatin modulating (e.g. modifying) agent contains an alkenyl or alkenylene group, cis (E) and trans (Z) isomerism may also occur. The present invention includes the individual stereoisomers of a chromatin modulating (e.g. modifying) agent and, where appropriate, the individual tautomeric forms thereof, together with mixtures thereof.

Separation of diastereoisomers or *cis*- and *trans*-isomers may be achieved by conventional techniques, *e.g.* by fractional crystallisation, chromatography or H.P.L.C. of a stereoisomeric mixture of an agent or a suitable salt or derivative thereof. An individual enantiomer of a chromatin modulating (e.g. modifying) agent may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid or base, as appropriate.

The present invention also encompasses all suitable isotopic variations of a chromatin modulating (e.g. modifying) agent or a pharmaceutically acceptable salt thereof. An isotopic variation of a chromatin modulating (e.g. modifying) agent or a pharmaceutically acceptable salt thereof is defined as one in which at least one atom is replaced by an atom having the same atomic number but an atomic mass different from the atomic mass usually found in nature. Examples of isotopes that may be incorporated into a chromatin modulating (e.g. modifying) agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of a chromatin modulating (e.g. modifying) agent and pharmaceutically acceptable salts thereof, for example, those in which a radioactive isotope such as ³H or ¹⁴C is incorporated are useful in drug and/or substrate tissue distribution studies. Tritiated, *i.e*., ³H, and carbon-14, *i.e.*, ¹⁴C, isotopes are particularly preferred for their ease of preparation and detectability. Further, substitution with isotopes such as deuterium, *i.e.*, ²H, may afford certain therapeutic advantages resulting from greater metabolic stability, for example, increased *in vivo* half-life or reduced dosage requirements and hence may be preferred in some circumstances. Isotopic variations of chromatin modulating (e.g. modifying) agents and pharmaceutically acceptable salts thereof can generally be prepared by conventional procedures using appropriate isotopic variations of suitable reagents.

It will be appreciated by those skilled in the art that a chromatin modulating (e.g. modifying) agent may be derived from a prodrug. Examples of prodrugs include entities that have certain protected group(s) and which may not possess pharmacological activity as such, but may, in certain instances, be administered (such as orally or parenterally) and thereafter metabolised in the body to form an agent of the present invention which are pharmacologically active.

It will be further appreciated that certain moieties known as "pro-moieties", for example as described in "Design of Prodrugs" by H. Bundgaard, Elsevier, 1985 (the disclosured of which is hereby incorporated by reference), may be placed on appropriate functionalities of chromatin modulating (e.g. modifying) agents. Such prodrugs are also included within the scope of the invention.

The present invention also includes the use of zwitterionic forms of a chromatin modulating (e.g. modifying) agent of the present invention. The terms used in the claims encompass one or more of the forms just mentioned.

### PHARMACEUTICALLY ACTIVE SALT

A chromatin modulating (e.g. modifying) agent may be administered as a pharmaceutically acceptable salt. Typically, a pharmaceutically acceptable salt may be readily prepared by using a desired acid or base, as appropriate. The salt may precipitate from solution and be collected by filtration or may be recovered by evaporation of the solvent.

### CHEMICAL SYNTHESIS METHODS

The chromatin modulating (e.g. modifying) agent may be prepared by chemical synthesis techniques.

It will be apparent to those skilled in the art that sensitive functional groups may need to be protected and deprotected during synthesis of a chromatin modulating (e.g. modifying) agent. This may be achieved by conventional techniques, for example as described in "Protective Groups in Organic Synthesis" by T W Greene and P G M Wuts, John Wiley and Sons Inc. (1991), and by P.J.Kocienski, in "Protecting Groups", Georg Thieme Verlag (1994).

It is possible during some of the reactions that any stereocentres present could, under certain conditions, be racemised, for example if a base is used in a reaction with a substrate having an having an optical centre comprising a base-sensitive group. This is possible during *e.g,* a guanylation step. It should be possible to circumvent potential problems such as this by choice of reaction sequence, conditions, reagents, protection/deprotection regimes etc. as is well-known in the art.

The compounds and salts may be separated and purified by conventional methods.

Separation of diastereomers may be achieved by conventional techniques, *e.g*. by fractional crystallisation, chromatography or H.P.L.C. of a stereoisomeric mixture of a compound or a suitable salt or derivative thereof. An individual enantiomer of a compound may also be prepared from a corresponding optically pure intermediate or by resolution, such as by H.P.L.C. of the corresponding racemate using a suitable chiral support or by fractional crystallisation of the diastereomeric salts formed by reaction of the corresponding racemate with a suitably optically active acid or base.

The chromatin modulating (e.g. modifying) agent or variants, homologues, derivatives, fragments or mimetics thereof may be produced using chemical methods to synthesise the chromatin modulating (e.g. modifying) agent in whole or in part. For example, if the chromatin modulating (e.g. modifying) agent is a peptide, then the peptide can be synthesized by solid phase techniques, cleaved from the resin, and purified by preparative high performance liquid chromatography (*e.g.*, Creighton (1983) Proteins Structures And Molecular Principles, WH Freeman and Co, New York NY). The composition of the synthetic peptides may be confirmed by amino acid analysis or sequencing (*e.g*., the Edman degradation procedure; Creighton, *supra).*

### CHEMICAL DERIVATIVE

The term "derivative" or "derivatised" as used herein includes chemical modification of an chromatin modulating (e.g. modifying) agent. Illustrative of such chemical modifications would be replacement of hydrogen by a halo group, an alkyl group, an acyl group or an amino group.

### CHEMICAL MODIFICATION

The chromatin modulating (e.g. modifying) agent may be a chemically modified agent.

The chemical modification of a chromatin modulating (e.g. modifying) agent may either enhance or reduce hydrogen bonding interaction, charge interaction, hydrophobic interaction, Van Der Waals interaction or dipole interaction.

In one aspect, the chromatin modulating (e.g. modifying) agent may act as a model (for example, a template) for the development of other compounds.

### PHARMACEUTICAL COMPOSITIONS

Pharmaceutical compositions useful in the present invention may comprise a therapeutically effective amount of chromatin modulating (e.g. modifying) agent(s) and pharmaceutically acceptable carrier, diluent or excipient (including combinations thereof).

Pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent may be selected with regard to the intended route of administration and standard pharmaceutical practice. Pharmaceutical compositions may comprise as - or in addition to - the carrier, excipient or diluent any suitable binder(s), lubricant(s), suspending agent(s), coating agent(s) or solubilising agent(s).

Preservatives, stabilizers, dyes and even flavoring agents may be provided in pharmaceutical compositions. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, pharmaceutical compositions useful in the present invention may be formulated to be administered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be administered by a number of routes.

Chromatin modulating (e.g. modifying) agents may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98155148.

If the chromatin modulating (e.g. modifying) agent is a protein, then said protein may be prepared *in situ* in the subject being treated. In this respect, nucleotide sequences encoding said protein may be delivered by use of non-viral techniques (*e.g*. by use of liposomes) and/or viral techniques (*e.g*. by use of retroviral vectors) such that the said protein is expressed from said nucleotide sequence.

### ADMINISTRATION

The present invention provides a method of modulating (e.g. modifying) chromatin structure in a subject comprising administering to the subject an effective amount of one or more chromatin modulating (e.g. modifying) agents identified according to the methods of the present invention.

The chromatin modulating (e.g. modifying) agents of the present invention may be administered alone but will generally be administered as a pharmaceutical composition comprising one or more components - e.g. when the components are in admixture with a suitable pharmaceutical excipient, diluent or carrier selected with regard to the intended route of administration and standard pharmaceutical practice.

For example, the components may be administered (*e.g.* orally) in the form of tablets, capsules, ovules, elixirs, solutions or suspensions, which may contain flavouring or colouring agents, for immediate-, delayed-, modified-, sustained-, pulsed- or controlled-release applications.

If the pharmaceutical is a tablet, then the tablet may contain excipients such as microcrystalline cellulose, lactose, sodium citrate, calcium carbonate, dibasic calcium phosphate and glycine, disintegrants such as starch (preferably com, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the chromatin modulating (e.g. modifying) agent may be combined with various sweetening or flavouring agents, colouring matter or dyes, with emulsifying and/or suspending agents and with diluents such as water, ethanol, propylene glycol and glycerin, and combinations thereof.

The routes for administration (delivery) include, but are not limited to, one or more of: oral (*e.g.* as a tablet, capsule, or as an ingestable solution), topical, mucosal (*e.g*. as a nasal spray or aerosol for inhalation), nasal, parenteral (*e.g.* by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, vaginal, epidural, sublingual.

It is to be understood that not all of the components of the pharmaceutical need be administered by the same route. Likewise, if the composition comprises more than one active component, then those components may be administered by different routes.

If a component is administered parenterally, then examples of such administration include one or more of: intravenously, intra-arterially, intraperitoneally, intrathecally, intraventricularly, intraurethrally, intrasternally, intracranially, intramuscularly or subcutaneously administering the component; and/or by using infusion techniques.

For parenteral administration, the component is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

As indicated, the component(s) useful in the present invention may be administered intranasally or by inhalation and is conveniently delivered in the form of a dry powder inhaler or an aerosol spray presentation from a pressurised container, pump, spray or nebuliser with the use of a suitable propellant, *e.g.* dichlorodifluoromethane, trichlorofluoromethane, dichlorotetrafluoroethane, a hydrofluoroalkane such as 1,1,1,2-tetrafluoroethane (HFA 134A^{™}) or 1,1,1,2,3,3,3-heptafluoropropane (HFA 227EA^{™}), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, *e.g*. using a mixture of ethanol and the propellant as the solvent, which may additionally contain a lubricant, *e.g.* sorbitan trioleate. Capsules and cartridges (made, for example, from gelatin) for use in an inhaler or insufflator may be formulated to contain a powder mix of the agent and a suitable powder base such as lactose or starch.

Alternatively, the component(s) may be administered in the form of a suppository or pessary, or it may be applied topically in the form of a gel, hydrogel, lotion, solution, cream, ointment or dusting powder. The component(s) may also be dermally or transdermally administered, for example, by the use of a skin patch. They may also be administered by the pulmonary or rectal routes. They may also be administered by the ocular route. For ophthalmic use, the compounds may be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the component(s) may be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it may be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The term "administered" also includes delivery by viral or non-viral techniques. Viral delivery mechanisms include but are not limited to adenoviral vectors, adeno-associated viral (AAV) vectos, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof.

### DOSE LEVELS

Typically, a physician will determine the actual dosage which will be most suitable for an individual subject. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

### FORMULATION

The component(s) may be formulated into a pharmaceutical composition, such as by mixing with one or more of a suitable carrier, diluent or excipient, by using techniques that are known in the art.

### GENERAL ASSAY TECHNIQUES

Any one or more appropriate targets - such as an amino acid sequence and/or nucleotide sequence of HSs - may be used for identifying a chromatin modulating (e.g. modifying) agent according to the present invention.

The target employed in such a test may be free in solution, affixed to a solid support, borne on a cell surface, or located intracellularly. The abolition of target activity or the formation of binding complexes between the target and the chromatin modulating (e.g. modifying) agent being tested may be measured.

The methods of the present invention may be a screen, whereby a number of chromatin modulating (e.g. modifying) agents are tested.

Techniques for drug screening may be based on the method described in Geysen, European Patent Application 84/03564, published on September 13, 1984. In summary, large numbers of different small peptide test compounds are synthesized on a solid substrate, such as plastic pins or some other surface. The peptide test compounds are reacted with a suitable target or fragment thereof and washed. Bound entities are then detected - such as by appropriately adapting methods well known in the art. A purified target may also be coated directly onto plates for use in a drug screening techniques. Alternatively, non-neutralising antibodies may be used to capture the peptide and immobilise it on a solid support.

It is expected that the methods of the present invention will be suitable for both small and large-scale screening of test compounds as well as in quantitative assays.

### IMMUNE MODULATOR

An immune modulator - such as a vaccine - may be identified using the methods of the present invention that is used for inoculation against cancer.

The immune modulator may be isolated from a suitable source, or it may be made synthetically or it may be prepared by the use of recombinant DNA techniques. The immune modulator may be administered in combination with an adjuvant to provide a generalised stimulation of the immune system.

### TREATMENT

It is to be appreciated that all references herein to treatment include one or more of curative, palliative and prophylactic treatments. Preferably, the term treatment includes at least curative treatment and/or palliative treatment.

The treatment may be for any disease such as cancer.

The treatment may be combined with other treatments such as radiotherapy.

### THERAPY

The agents may be used as therapeutic agents - *i.e.* in therapy applications.

As with the term "treatment", the term "therapy" includes curative effects, alleviation effects, and prophylactic effects.

The therapy may be on humans or animals.

The therapy can include the treatment of diseases such as cancer.

### ANIMAL TEST MODELS

*In vivo* models may be used to investigate and/or design therapies or therapeutic chromatin modulating (e.g. modifying) agents to treat disease such as cancer. The models could be used to investigate the effect of various tools/lead compounds on a variety of parameters, which are implicated in the development of or treatment of a cancer. The animal test model will be a non-human animal test model.

### FUSION PROTEINS

An amino acid sequence for use in the present invention may be produced as a fusion protein, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence to allow removal of fusion protein sequences. Preferably the fusion protein will not hinder the activity of the protein sequence.

The fusion protein may comprise an antigen or an antigenic determinant fused to the substance of interest. The fusion protein may be a non-naturally occurring fusion protein comprising a substance, which may act as an adjuvant in the sense of providing a generalised stimulation of the immune system. The antigen or antigenic determinant may be attached to either the amino or carboxy terminus of the substance.

### EXPRESSION VECTORS

A nucleotide sequence may be incorporated into a recombinant replicable vector. In one aspect of the present invention, the nucleotide sequence may encode a fragmenting means. In another aspect, the nucleotide sequence may encode a chromatin modulating (e.g. modifying) agent. The vector may be used to replicate and express the nucleotide sequence. Expression may be controlled using control sequences, which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The protein produced by a host recombinant cell or tissue by expression of a nucleotide sequence may be secreted or may be contained intracellularly depending on the sequence and/or the vector used. The coding sequences can be designed with signal sequences, which direct secretion of the substance coding sequences through a particular prokaryotic or eukaryotic cell membrane.

### HYBRIDISATION

As used herein, the term "hybridisation refers to "the process by which a strand of nucleic acid joins with a complementary strand through base pairing" as well as the process of amplification as carried out in polymerase chain reaction (PCR) technologies.

The present invention encompasses the use of nucleotide sequences that are capable of hybridising to nucleotide sequences of the present invention, or any fragment, variant or homologue thereof.

### VARIANTS/HOMOLOGUES/DERIVATIVES

The present invention encompasses the use of variants, homologues and derivatives of nucleotide and amino acid sequences. Here, the term "homologue" means an entity having a certain homology with amino acid sequences or nucleotide sequences. Here, the term "homology" can be equated with "identity".

In the present context, an homologous sequence is taken to include an amino acid sequence which may be at least 75, 85 or 90% identical, preferably at least 95 or 98% identical to the subject sequence. Although homology can also be considered in terms of similarity (i.e. amino acid residues having similar chemical properties/functions), it is preferred to express homology in terms of sequence identity.

An homologous sequence is taken to include a nucleotide sequence which may be at least 75, 85 or 90% identical, preferably at least 95 or 98% identical to the subject sequence.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al.*, 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel *et al*., 1999 ibid, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8)

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Homologous substitution (substitution and replacement are both used herein to mean the interchange of an existing amino acid residue, with an alternative residue) may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyprotine^{#} L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. Such modifications may be carried out to enhance the *in vivo* activity or life span of nucleotide sequences.

### REGULATORY SEQUENCES

In some applications, polynucleotides may be linked to a regulatory sequence which is capable of providing for the expression of the coding sequence, such as by a chosen cell. By way of example, the present invention covers a vector comprising operably linked to such a regulatory sequence, *i.e.* the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, *e.g.* an RNA polymerase binding site.

Enhanced expression of polypeptides may be achieved by the selection of heterologous regulatory regions, *e.g*. promoter, secretion leader and terminator regions, which serve to increase expression and, if desired, secretion levels of the protein of interest from the chosen expression host and/or to provide for the inducible control of expression.

Aside from the promoter native to the nucleotide sequence encoding the polypeptide, other promoters may be used to direct expression of the polypeptide. The promoter may be selected for its efficiency in directing the expression of the polypeptide in the desired expression host.

In another embodiment, a constitutive promoter may be selected to direct the expression of the polypeptide. Such an expression construct may provide additional advantages since it circumvents the need to culture the expression hosts on a medium containing an inducing substrate.

Examples of strong constitutive and/or inducible promoters which are preferred for use in fungal expression hosts are those which are obtainable from the fungal nucleotide sequences for xylanase (*xlnA*), phytase, ATP-synthetase, subunit 9 (*oliC*), triose phosphate isomerase (*tpi*), alcohol dehydrogenase *(AdhA),* α-amylase *(amy),* amyloglucosidase (AG - from the g/aA nucleotide sequence), acetamidase *(amdS)* and glyceraldehyde-3-phosphate dehydrogenase (*gpd*) promoters.

Examples of strong yeast promoters are those obtainable from the nucleotide sequences for alcohol dehydrogenase, lactase, 3-phosphoglycerate kinase and triosephosphate isomerase.

Examples of strong bacterial promoters are the α-amylase and *SP02* promoters as well as promoters from extracellular protease nucleotide sequences.

Hybrid promoters may also be used to improve inducible regulation of the expression construct.

The promoter can additionally include features to ensure or to increase expression in a suitable host. For example, the features can be conserved regions such as a Pribnow Box or a TATA box. The promoter may even contain other sequences to affect (such as to maintain, enhance, decrease) the levels of expression of a nucleotide sequence. Suitable other sequences include the Sh1-intron or an ADH intron. Other sequences include inducible elements - such as temperature, chemical, light or stress inducible elements. Also, suitable elements to enhance transcription or translation may be present. An example of the latter element is the TMV 5' signal sequence (see Sleat Gene 217 [1987] 217-225; and Dawson Plant Mol. Biol. 23 [1993] 97).

### SECRETION

It may be desirable for a polypeptide to be secreted from the expression host into the culture medium from where the polypeptide may be more easily recovered.

Typical examples of heterologous secretion leader sequences are those originating from the fungal amyloglucosidase (AG) nucleotide sequence (*gla*A - both 18 and 24 amino acid versions *e.g*. from *Aspergillus*)*,* the a-factor nucleotide sequence (yeasts *e.g. Saccharomyces* and *Kluyveromyces)* or the α-amylase nucleotide sequence *(Bacillus).*

### CONSTRUCTS

Nucleotide sequences may be present in a construct.

The term "construct" - which is synonymous with terms such as "conjugate", "cassette" and "hybrid" - includes a nucleotide sequence directly or indirectly attached to a promoter. An example of an indirect attachment is the provision of a suitable spacer group such as an intron sequence, such as the Sh1-intron or the ADH intron, intermediate to the promoter and the nucleotide sequence of the present invention. The same is true for the term "fused" which includes direct or indirect attachment. In some cases, the terms do not cover the natural combination of the nucleotide sequence coding for the protein ordinarily associated with the wild type nucleotide sequence promoter and when they are both in their natural environment.

The construct may even contain or express a marker, which allows for the selection of the nucleotide sequence construct in, for example, a bacterium, preferably of the genus Bacillus, such as *Bacillus subtilis,* or plants into which it has been transferred. Various markers exist which may be used, such as for example those encoding mannose-6-phosphate isomerase (especially for plants) or those markers that provide for antibiotic resistance - *e.g*. resistance to G418, hygromycin, bleomycin, kanamycin and gentamycin.

### VECTORS

Nucleotide sequences may be present in a vector.

The term "vector" includes expression vectors and transformation vectors and shuttle vectors.

The term "expression vector" means a construct capable of *in vivo* or *in vitro* expression.

The term "transformation vector" means a construct capable of being transferred from one entity to another entity - which may be of the species or may be of a different species. If the construct is capable of being transferred from one species to another - such as from an *E*. *coli* plasmid to a bacterium, such as of the genus Bacillus, then the transformation vector is sometimes called a "shuttle vector". It may even be a construct capable of being transferred from an *E*. *coli* plasmid to an Agrobacterium to a plant

The vectors may be transformed into a suitable host cell as described below to provide for expression of a polypeptide.

The vectors may be for example, plasmid, virus or phage vectors provided with an origin of replication, optionally a promoter for the expression of the said polynucleotide and optionally a regulator of the promoter.

The vectors may contain one or more selectable marker nucleotide sequences. The most suitable selection systems for industrial micro-organisms are those formed by the group of selection markers which do not require a mutation in the host organism. Examples of fungal selection markers are the nucleotide sequences for acetamidase (*amdS*), ATP synthetase, subunit 9 (*oliC*), orotidine-5'-phosphate-decarboxylase (*pvrA*), phleomycin and benomyl resistance (benA). Examples of non-fungal selection markers are the bacterial G418 resistance nucleotide sequence (this may also be used in yeast, but not in filamentous fungi), the ampicillin resistance nucleotide sequence (*E. coli*), the neomycin resistance nucleotide sequence *(Bacillus)* and the *E. coli uid*A nucleotide sequence, coding for β-glucuronidase (GUS).

Vectors may be used *in vitro,* for example for the production of RNA or used to transfect or transform a host cell.

Thus, polynucleotides may be incorporated into a recombinant vector (typically a replicable vector), for example a cloning or expression vector. The vector may be used to replicate the nucleic acid in a compatible host cell.

Genetically engineered host cells may be used for expressing an amino acid sequence (or variant, homologue, fragment or derivative thereof) in screening methods for the identification of chromatin modulating (e.g. modifying) agents. Such engineered host cells could be used to screen peptide libraries or organic molecules. The chromatin modulating (e.g. modifying) agents of said amino acid sequences, such as antibodies, peptides or small organic molecules will provide the basis for pharmaceutical compositions for the treatment of cancer. Such agents may be administered alone or in combination with other therapeutics such as radiotherapy, for the treatment of disease such as cancer.

### SCREENING METHODS

A plurality of candidate compounds may be screened using the methods described below. In particular, these methods may be suited for screening libraries of compounds.

Where the candidate compounds are proteins, in particular antibodies or peptides, libraries of candidate compounds can be screened using phage display techniques. Phage display is a protocol of molecular screening which utilises recombinant bacteriophage. The technology involves transforming bacteriophage with a gene that encodes the library of candidate compounds, such that each phage or phagemid expresses a particular candidate compound. The transformed bacteriophage (which preferably is tethered to a solid support) expresses the appropriate candidate compound and displays it on their phage coat. Specific candidate compounds which are capable of interacting with hr44 are enriched by selection strategies based on affinity interaction. The successful candidate agents are then characterised. Phage display has advantages over standard affinity ligand screening technologies. The phage surface displays the candidate agent in a three dimensional configuration, more closely resembling its naturally occurring conformation. This allows for more specific and higher affinity binding for screening purposes.

The yeast two-hybrid system described above can be used to screen for polypeptides. For example, a human cDNA from a tissue may be substituted with a cDNA library from a different tissue or species, or a combinatorial library of synthetic oligonucleotides.

Another method of screening a library of compounds utilises eukaryotic or prokaryotic host cells which are stably transformed with recombinant DNA molecules expressing the library of compounds. Such cells, either in viable or fixed form, can be used for standard binding-partner assays. See also Parce et al. (1989) Science 246:243-247; and Owicki et al. (1990) Proc. Nat'l Acad. Sci. USA 87;4007-4011, which describe sensitive methods to detect cellular responses. Competitive assays are particularly useful, where the cells expressing the library of compounds are incubated with a labelled antibody, such as ¹²⁵I-antibody, and a test sample such as a candidate compound whose binding affinity to the binding composition is being measured. The bound and free labelled binding partners are then separated to assess the degree of binding. The amount of test sample bound is inversely proportional to the amount of labelled antibody bound.

Any one of numerous techniques can be used to separate bound from free binding partners to assess the degree of binding. This separation step could typically involve a procedure such as adhesion to filters followed by washing, adhesion to plastic following by washing, or centrifugation of the cell membranes.

Still another approach is to use solubilized, unpurified or solubilized purified protein either extracted from expressing mammalian cells or from transformed eukaryotic or prokaryotic host cells. This allows for a "molecular" binding assay with the advantages of increased specificity, the ability to automate, and high drug test throughput.

Another technique for candidate compound screening involves an approach which provides high throughput screening for new compounds having suitable binding affinity and is described in detail in International Patent application no. WO 84/03564 (Commonwealth Serum Labs.), published on September 13 1984. First, large numbers of different small peptide test compounds are synthesised on a solid substrate, *e.g.*, plastic pins or some other appropriate surface; see Fodor *et al.* (1991). Then all the pins are reacted with solubilized protein and washed. The next step involves detecting bound protein. Detection may be accomplished using a monoclonal antibody to the protein of interest. Compounds which interact specifically with the protein may thus be identified.

Rational design of candidate compounds likely to be able to interact with the protein may be based upon structural studies of the molecular shapes of the protein and/or its *in vivo* binding partners. One means for determining which sites interact with specific other proteins is a physical structure determination, *e.g.*, X-ray crystallography or two-dimensional NMR techniques. These will provide guidance as to which amino acid residues form molecular contact regions. For a detailed description of protein structural determination, see, *e.g*., Blundell and Johnson (1976) Protein Crystallography, Academic Press, New York.

### SCREENING FOR COMPOUNDS WHICH MODULATE THE ACTIVITY OF A PROTEIN

As mentioned above, the compound may modulate the capacity of a protein to interact with an *in vivo* binding partner. Once the *in vivo* binding partner has been identified, there are a number of methods known in the art by which compounds may be screened for their capacity to modulate the interaction between the protein and its binding partner, or the physiological effect of the interaction.

For example, *in vitro* competitive binding assays using either immobilised protein or binding partner (see above) can be used to investigate the capacity of a library of test compounds to inhibit or enhance the protein:binding partner interaction.

Alternatively, the yeast two-hybrid system may be used to identify compounds which affect the protein:binding partner interaction. Compounds that increase or decrease reporter expression relative to a user-defined threshold (for example, a five-fold increase or five-fold decrease) are thus identified as being modulators of the interaction.

### SCREENING FOR COMPOUNDS WHICH MODULATE THE EXPRESSION OF A PROTEIN

There are numerous methods suitable for measuring the expression of a protein, by measuring expression of the gene or the protein.

Gene expression may be measured using the polymerase chain reaction (PCR), for example using RT-PCR. RT-PCR may be a useful technique where the candidate compound is designed to block the transcription of a gene. Alternatively, the presence or amount of mRNA can be detected using Northern blot. Northern blotting techniques are particularly suitable if the candidate compound is designed to act by causing degradation of the mRNA.

Protein expression may be detected or measures by a number of known techniques, including Western blotting, immunoprecipitation, immunocytochemisty techniques, immunohistochemistry, *in situ* hybridisation, ELISA, radio-immunolabelling, fluorescent labelling techniques (fluorimetry, confocal microscopy) and spectrophotometry.

### HOST CELLS

As used herein, the term "host cell" refers to any cell that comprises the fragmenting means and/or chromatin modulating (e.g. modifying) agent and/or can be used to express the fragmenting means and/or chromatin modulating (e.g. modifying) agent according to the present invention (if said agent is a polynucleotide).

Thus, a further embodiment of the present invention provides host cells transformed or transfected with a polynucleotide that is or expresses the fragmenting means and/or chromatin modulating (e.g. modifying) agent of the present invention. Preferably said polynucleotide is carried in a vector for the replication and expression of polynucleotides that are to be the chromatin modulating (e.g. modifying) agents or are to express the fragmenting means and/or chromatin modulating (e.g. modifying) agent The cells will be chosen to be compatible with the said vector and may for example be prokaryotic (for example bacterial), fungal, yeast or plant cells.

The gram-negative bacterium *E*. *coli* is widely used as a host for heterologous nucleotide sequence expression. However, large amounts of heterologous protein tend to accumulate inside the cell. Subsequent purification of the desired protein from the bulk of *E*. *coli* intracellular proteins can sometimes be difficult.

In contrast to *E. coli*, bacteria from the genus Bacillus are very suitable as heterologous hosts because of their capability to secrete proteins into the culture medium. Other bacteria suitable as hosts are those from the genera Streptomyces and Pseudomonas.

Depending on the nature of the polynucleotide and/or the desirability for further processing of the expressed protein, eukaryotic hosts such as yeasts or other fungi may be preferred. In general, yeast cells are preferred over fungal cells because yeast cells are easier to manipulate. However, some proteins are either poorly secreted from the yeast cell, or in some cases are not processed properly (*e.g.* hyperglycosylation in yeast). In these instances, a different fungal host organism should be selected.

Examples of expression hosts are fungi such as Aspergillus species (such as those described in EP-A-0184438 and EP-A-0284603) and Trichoderma species; bacteria such as Bacillus species (such as those described in EP-A-0134048 and EP-A-0253455), Streptomyces species and Pseudomonas species; and yeasts such as Kluyveromyces species (such as those described in EP-A-0096430 and EP-A-0301670) and Saccharomyces species. By way of example, typical expression hosts may be selected from *Aspergillus niger, Aspergillus niger var. tubigenis, Aspergillus niger var. awamori, Aspergillus aculeatis, Aspergillus nidulans, Aspergillus orvzae, Trichoderma reesei, Bacillus subtilis, Bacillus licheniformis, Bacillus amyloliquefaciens, Kluyveromyces lactis* and *Saccharomyces cerevisiae.*

The use of host cells - such as yeast, fungal and plant host cells - may provide for post-translational modifications (*e.g*. myristoylation, glycosylation, truncation, lapidation and tyrosine, serine or threonine phosphorylation) as may be needed to confer optimal biological activity on recombinant expression products of the present invention.

### TRANSFORMATION

Teachings on the transformation of cells are well documented in the art, for example see Sambrook et al (Molecular Cloning: A Laboratory Manual, 2nd edition, 1989, Cold Spring Harbor Laboratory Press) and Ausubel et al., Current Protocols in Molecular Biology (1995), John Wiley & Sons, Inc.

If a prokaryotic host is used then the nucleotide sequence may need to be suitably modified before transformation - such as by removal of introns.

A review of the principles of heterologous nucleotide sequence expression in *Saccharomyces cerevisiae* and secretion of nucleotide sequence products is given by E Hinchcliffe E Kenny (1993, "Yeast as a vehicle for the expression of heterologous nucleotide sequences", Yeasts, Vol 5, Anthony H Rose and J Stuart Harrison, eds, 2nd edition, Academic Press Ltd.).

A host cell may be transformed with a fragmenting means or a chromatin modulating (e.g. modifying) agent. Host cells transformed with the fragmenting means or a chromatin modulating (e.g. modifying) agent may be cultured under conditions suitable for the expression of the encoded protein. The protein produced by a recombinant cell may be expressed within the cell or even displayed on the surface of the cell. If desired, and as will be understood by those of skill in the art, expression vectors containing coding sequences can be designed with signal sequences which direct secretion of the coding sequences through a particular prokaryotic or eukaryotic cell membrane. Other recombinant constructions may join the coding sequence to nucleotide sequence encoding a polypeptide domain which will facilitate purification of soluble proteins (Kroll DJ et al (1993) DNA Cell Biol 12:441-53).

The fusion protein may comprise an antigen or an antigenic determinant. In this embodiment, the fusion protein may be a non-naturally occurring fusion protein comprising a substance which may act as an adjuvant in the sense of providing a generalised stimulation of the immune system. The antigen or antigenic determinant may be attached to either the amino or carboxy terminus.

In another embodiment of the invention, the amino acid sequence may be ligated to a heterologous sequence to encode a fusion protein. For example, for screening of peptide libraries for chromatin modulating (e.g. modifying) agents.

### GENERAL EXPERIMENTAL

Methods are described that are capable of implementing detection, mapping and analysis of HSs on a genome-wide scale. The early steps of both procedures are identical, but the subsequent strategies employed for analysing the HS-induced DNA cleavage fragments differ.

### Nuclease cleavage of HSs

Almost all of the original studies have been based on the use of the sequence-nonspecific nucleases (such as DNAase I and micrococcal nuclease), but it is also possible to use sequence-specific restriction enzymes to introduce specific cuts into HSs. These enzymes cut HSs in a more selective manner because they depend on a particular 4-8 nucleotide target sequence motif being present in the exposed DNA region of an HS. This feature renders the use of restriction enzymes rather unsuitable for a detailed study of particular HSs, but is not a limitation for a global analysis method were a 'tuneable' degree of selectivity is rather advantageous. The use of the restriction enzyme Hinfl for mapping local HSs of the chicken lysozyme gene has been reported (see e.g. Gross and Garrard, 1988; Kontaraki *et al.*, 2000), but only as part of an armoury of additional nucleases to demonstrate the presence of HSs previously mapped by other means. In this invention a range of restriction nucleases with different target site specificities are used for an entirely different purpose and thus represent a novel use of these enzymes. By applying restriction enzymes it becomes possible to cleave the genomic DNA exposed in HSs in a manner that produces a manageable number of genomic DNA fragments that can be subsequently detected and analysed. The restriction enzyme can be applied to purified preparations of eukaryotic nuclei. Alternatively, the enzyme can also be introduced into cells under *in vivo* conditions by using cell permeabilization techniques (e.g. Kontaraki *et al.,* 2000), or (at least in principle) by expressing the restriction nuclease from DNA expression constructs introduced into cells by established techniques.

### Global Detection of Restriction Enzyme-cleaved HSs

The restriction enzyme treatment introduces a number of sequence-specific cuts into genomic DNA at sites that are exposed to the nuclease because they are present in an HS configuration. There are two techniques outlined below that convert this cutting pattern into a specific 'fingerprint' that can be detected, archived and analysed by the GACT methods.

### Restriction Landmark Genomic Scanning ('RLGS')

This method is a two-dimensional gel electrophoresis method that was first described almost a decade ago (Hatada *et a*/*.,* 1991), but has not become widely applied due to its specialized nature. The only applications proposed by authors were the application of RLGS for the detection of genomic amplifications/deletions and for genetic studies using polymorphic spots. The RLGS concept has been recently applied in a study to visualize different global CpG island methylation patterns in different tumor types (Costello *et al.*, 2000). The GACT method proposed here combines some essential ideas from the two concepts described above and introduces a number of additional modifications to construct a workable technique for the experimental analysis of global chromatin topology. The established method for visualizing HSs is by a method called 'indirect endlabelling' (Wu, 1980), but this technique only gives results for one particular region of the genome (typically spanning 1-5 kb). In contrast, GACT is designed the detection and comparison of several thousand sites in a single experiment.

In a typical experiment cells are temporarily permeabilised by lysolecithin treatment and then incubated in a buffer containing a specific restriction enzyme. This endonuclease enters the cell and cleaves DNA target sites that are exposed in HSs. Other target sites that are present in conventional chromatin configurations are not affected under these circumstances. After allowing time for cleavage (typically 30-60 minutes) the cells are permeabilised again and incubated with DNA polymerase (Klenow fragment or 'Sequenase') and at least one radioactively labelled dNTP. This step leads to the selective incorporation of the radioisotope into the genomic DNA at the sites that have been cleaved by the restriction enzyme (i.e. DNA present in HSs gets labelled). After 30-60 minutes the cells are lysed and the genomic DNA is purified from them by established methods. The DNA is then cleaved with a second restriction enzyme (typically with a 6 bp recognition sequence) to reduce the average size of the fragments. The fragment mixture is then subjected to RLGS (which typically includes an in-gel digest with a 4 bp cutting restriction enzyme) to produce a two-dimensional gel image. The labelled fragments are subsequently analysed by methods - such as phospho-imaging and/or autoradiography. Each detectable spot on the two-dimensional gel image corresponds to a cleaved HS, and therefore the entire gel image represents a global map of the chromatin topology present in the original cell sample.

The number of HSs analysed per gel will typically depend on a number of different parameters, including:
- The physical dimensions of the two-dimensional gel: larger gels will provide improved resolution and thus reveal larger numbers of HSs
- The nature and concentration of the gel matrices and buffers used for the electrophoresis steps: short DNA fragments will be better resolved on high percentage gels, whereas longer fragments need to be resolved in low percentage gels for optimal separation
- The choice of restriction enzymes used for carrying out the different fragmentation steps as outlined above: The use of restriction enzymes with a 4 bp recognition sequence during the HS cleavage reaction will result in a larger number of HSs being cleaved and thus have a direct effect on the number of spots displayed in the two-dimensional gel

In addition to utilizing a simple spot scoring method (e.g. spot present or absent) for analysing the results of the method, it is likely that some spots will be display an intermediate signal level, indicating that the corresponding HSs are differentially accessible in different cells. This could either reflect a heterogeneity in the cell population used for the assay (e.g. presence of different cell types), or otherwise indicate the presence of HSs that are undergoing transitions in their structure due to normal physiological processes. The proposed technique therefore is capable of providing a large number of data points that reflect quantitatively the states of thousands of HSs in a single experiment.

Generation of a Distinct HS Profile using a Polymerase Chain Reaction (PCR)-based Approach

The two-dimensional gel method outlined above is very suitable for generating a distinct pattern that reflects the presence of HSs on a genome-wide level in a quantitative manner. For applications of GATC in high-throughput screening applications it will, however, be essential to develop an alternative approach that can be automated in its entirety. The GACT strategy can be modified to avoid the RLGS step described above by using a novel PCR approach.

In a typical experiment the DNA present in a HS configuration will be cleaved as described above with a restriction enzyme. Instead of labelling the cut site, an 'adapter' oligonucleotide is added that contains a single-stranded region which is complementary to the 'sticky' end produced by the restriction nuclease (to maximize the region of hybridisation between the cut end and the adapter oligonucleotide, restriction nucleases SauIIIA, DpnII, Mbol, NlaIII and Ts5091 will typically be the enzymes of choice). The adapter oligonucleotide is then covalently linked to the cleaved sequence in the genomic DNA with DNA ligase. This step results in the specific addition of a short stretch of known sequence to each DNA sequence that is exposed as a HS. The genomic DNA is subsequently extracted and purified. Any unincorporated adapter oligonucleotides (or homodimers formed during the ligation reaction) are removed by size-exclusion chromatography ('spin columns'). The DNA fragment to which the adapter oligonucleotide has been specifically ligated under the described conditions can then be selectively analysed by a polymerase chain reaction (PCR) step using a particular DNA primer combination. One of the primers will be complementary to the sequence of the adapter oligonucleotide and will be fluorescently labeled. The other primer will be a short oligonucleotide (around 5-8 nucleotides) of arbitrary sequence. When the PCR reaction is carried out under 'low stringency' conditions (i.e. low annealing temperatures, typically ranging between 45-55°C) a subset of the genomic DNA will be specifically amplified and result in the formation of discrete DNA fragments containing an incorporated fluorescent label. This approach shares certain technical similarities with the 'differential display' technique (Liang and Pardee, 1992), but is conceptually distinct and fulfils a different purpose.

The resulting PCR products are subsequently separated by size on a fluorescent DNA analysis system similar to the ones currently in use for automated DNA sequencing. The procedure results in a specific one-dimensional 'band' pattern that reflects a subset of cleaved HSs that is originally present in the sample. Different and non-overlapping HS subsets can be revealed by carrying out the PCR reactions using different arbitrary oligonucleotides in presence of the labelled adapter-specific primer.

To improve the efficiency of the procedure, the different primer combinations can be labelled with different fluorescent labels so that the resulting PCR products can be analysed within a single capillary of the fluorescent DNA analysis system. All these steps can be implemented using robotic systems and thus ensure complete automation.

Identification of HSs Revealed by GACT Profiling

The methods describe two independent means of generating specific 'fingerprints' that reflect the presence of a large proportion of HSs present within the genomes of a test cell population. While this already constitutes considerable progress in comparison with the currently available technology, it is also important to provide means of identifying the genomic origin of individual spots or bands that generate these profiles. It then becomes possible to link the measured presence/absence of detected HSs to particular genes, and thus provide information about the biological events that are controlled through changes in chromatin topology. The epigenomic data obtained from GACT will provide a significant amount of new insights because it allows a direct link between information about tissue- and stage specific HSs and specific genomic regions and therefore results in specifically annotated genomic sequence data. Such annotation schemes could be improved further by the addition of cross-species epigenomic data (e.g. based on the systematic mapping of HSs in the mouse genome) where it will be possible to look at a range of different tissues and endogenous cell types much more readily. The GACT information will complement data obtained from other global analysis technologies, such as gene expression patterns based on DNA microarray and proteome projects.

The PCR-based approach outlined above is ideally suited for developing such a link between the profile generated and the genomic origin of the HSs detected. Each band corresponds to a DNA fragment that has been specifically amplified by two distinct primer oligonucleotides. It is thus possible to elute various fragments from the gel matrix and to amplify the DNA fragments present in order to obtain sufficient amounts for DNA sequence analysis. A systematic application of this technique to a large number of PCR fragments will result in a substantial sequence documentation of the amplified DNA fragments. Once fragments of a particular size are identified and sequenced, the resulting information can be used in all subsequent experiments that use the same primer combinations and PCR amplification conditions. It is thus possible to build up extensive databases that allow a direct link between the HS mapping data and genomic DNA sequences.

### FIGURES

The present invention will now be described by way of example, in which reference is made to the following Figures:
Figure 1, which presents a series of diagrams;
Figure 2, which presents a series of photographs;
Figure 3, which present a series of diagrams;
Figure 4, which presents a series of diagrams;
Figure 5, which presents a series of diagrams;
Figure 6, which presents a series of diagrams;
Figure 7, which presents a series of photographs; and
Figure 8, which represents a table.

In more detail:

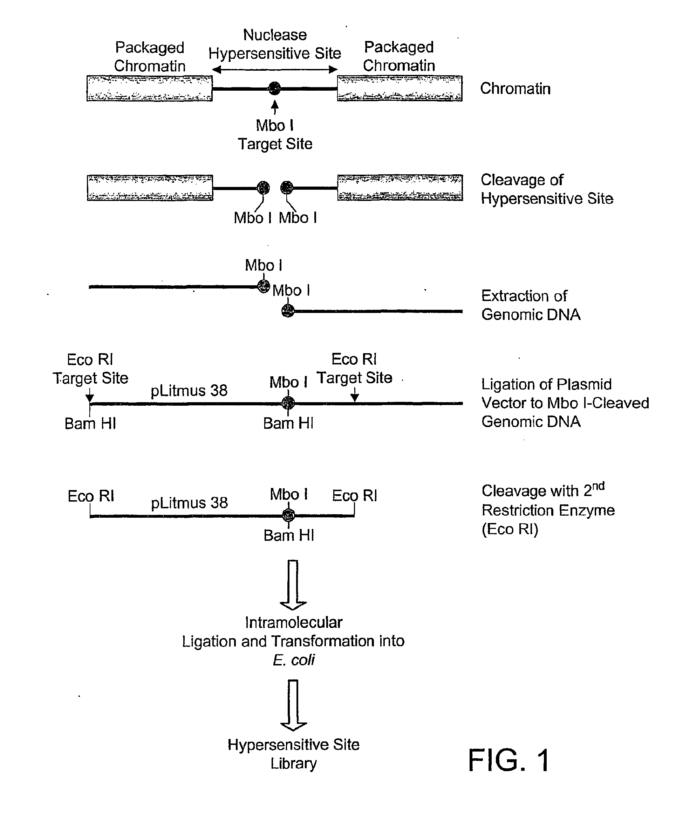

Figure 1 represents a schematic diagram of the principle of large-scale mapping of genomic regulatory motifs in HeLa cells using a hypersensitive site cloning strategy.

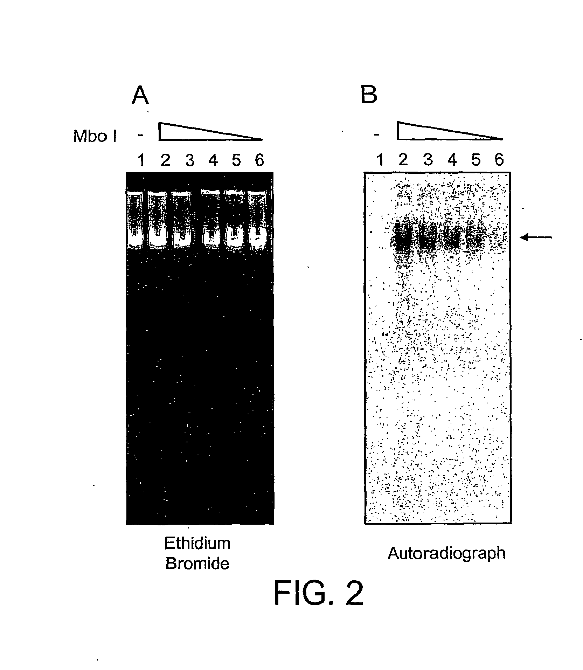

Figure 2 represents series of photographs. Figure 2A represents a 0.7 % agarose gel in which genomic DNA is exposed to various amounts of Mbol. Lane 1 = no enzyme (negative control); Lane 2 = 100 units Mbo I; Lane 3 = 50 units Mbol; Lane 4 = 25 units Mbo I; Lane 5 = 10 units Mbo I; Lane 6 = 5 units Mbo I. Figure 2B shows the results of monitoring the extent of specific Mbol cleavage more quantitatively using a ³²P-labeled adapter containing a BamHI 'sticky' end ligated to the samples.

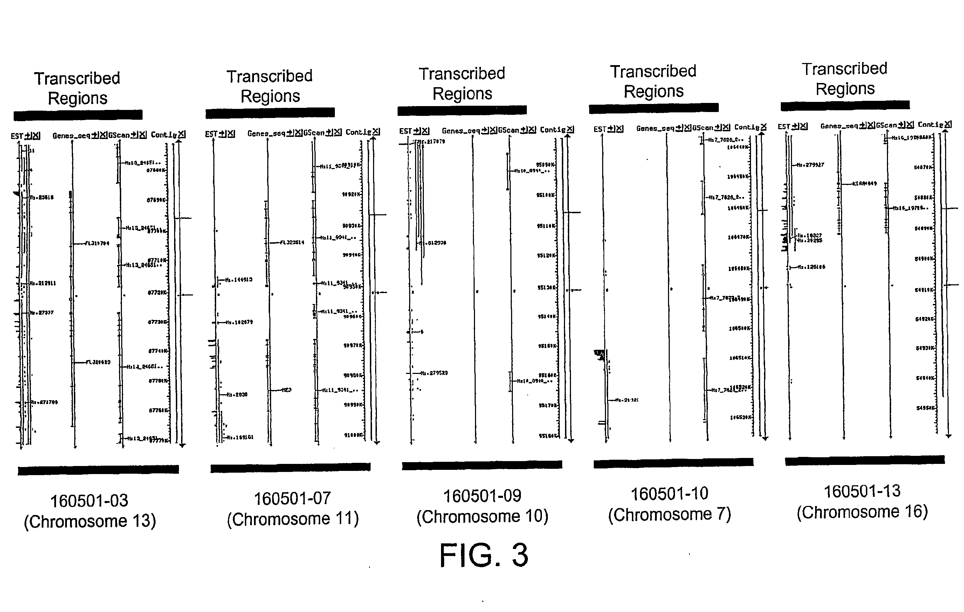

Figure 3 represents a series of diagrams illustrating the positions of mapped sites of a selection of five randomly chosen HS clones. The location of the Mbol site identified in the BLAST search is indicated by a red square near the centre of each diagram. The positions of the mapped sites are compatible with their potential role as transcriptional regulatory motifs, such as enhancers and insulators.

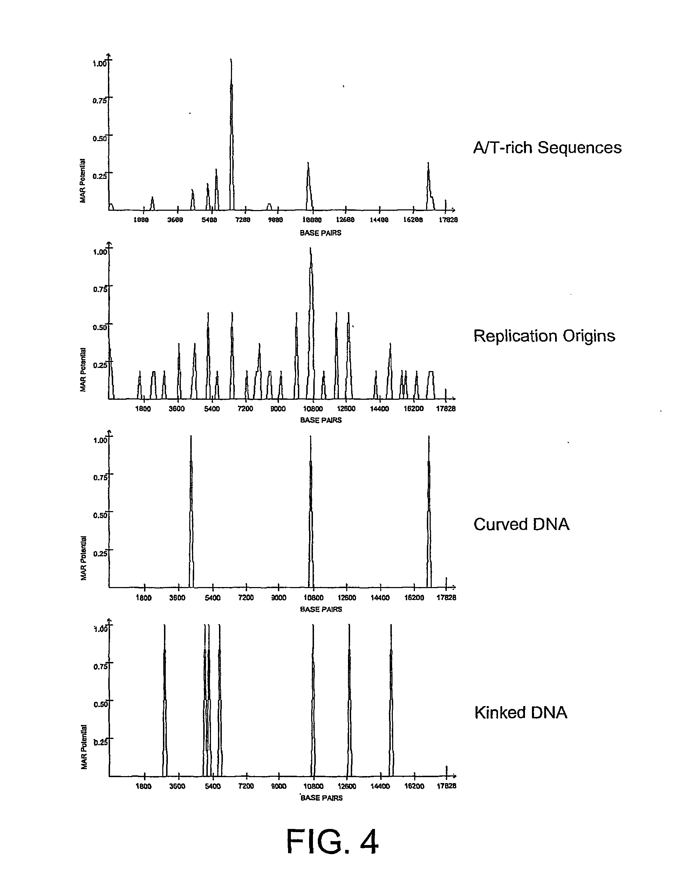

Figure 4 which presents a series of diagrams illustrates that several HS clones contain sequences compatible with replication consensus motifs, curved or kinked DNA sequences.

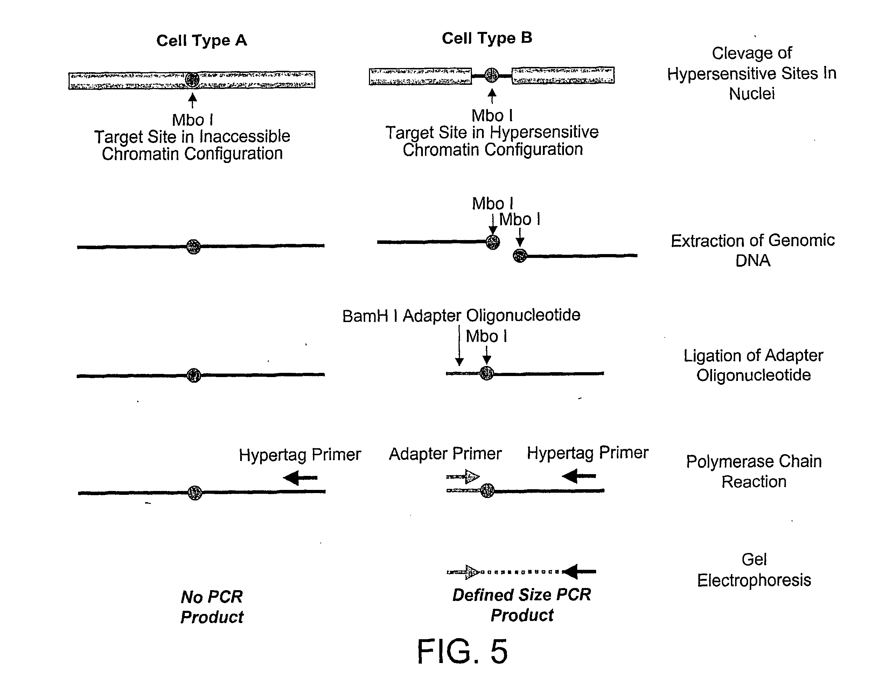

Figure 5 which represents a schematic diagram of the principle of hypertag display illustrates that the method may be used to compare the state of a particular HS in various cell types.

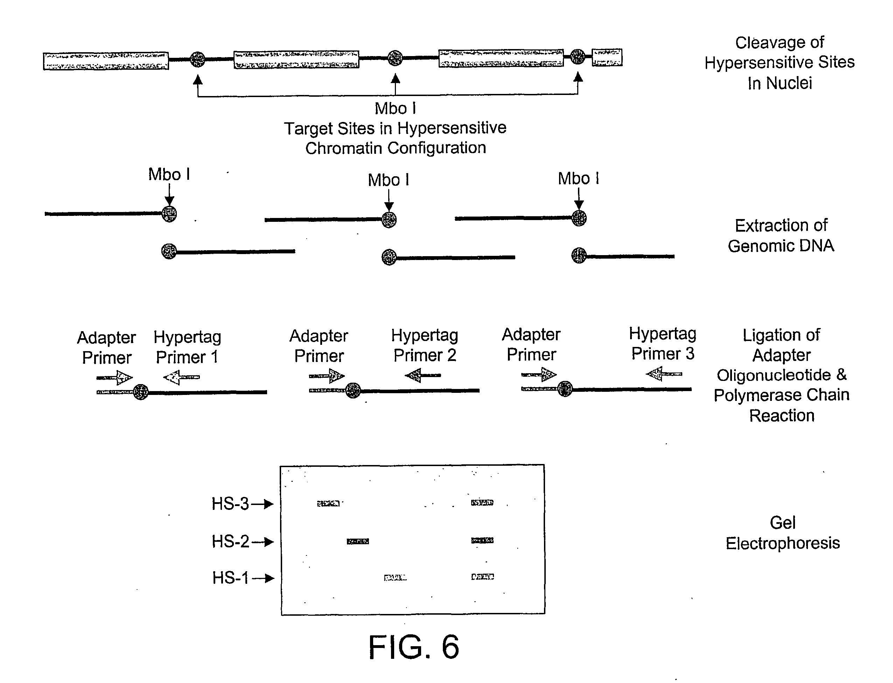

Figure 6 which represents a schematic diagram illustrates that hypertag display can be adapted to the detection of several different HSs within a sample by designing the hypertag primers to yield amplification products of different sizes.

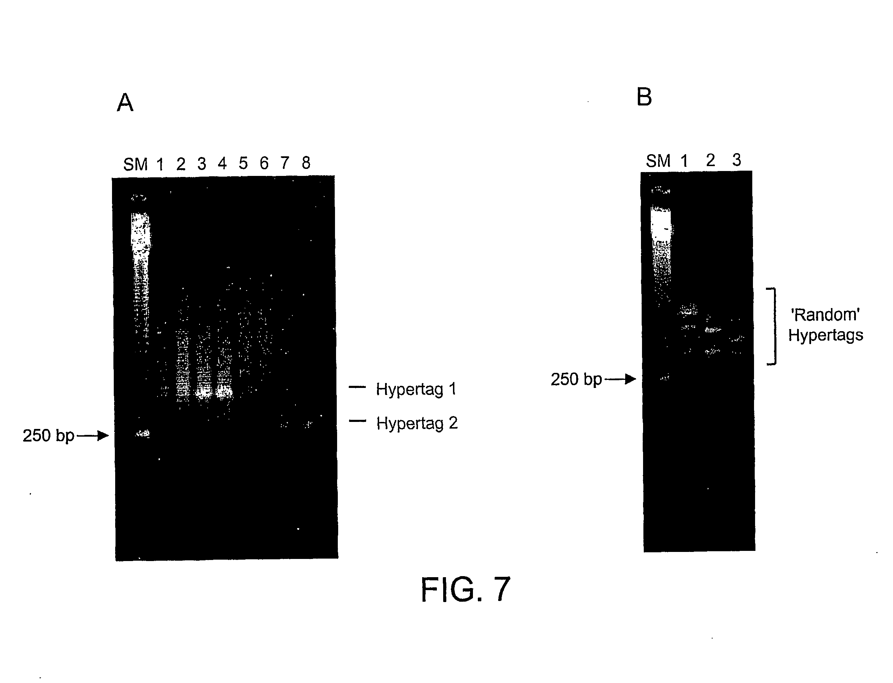

Figure 7 which represents a series of photographs illustrates the hypertag amplification products obtained. (A) Duplicate DNA samples from untreated nuclei (No Mbol added; lanes 1-2 and 5-6) and Mbol-HS cleaved DNA (50 Units Mbol; lanes 3-4 and 7-8) were amplified with 'Hypertag Primer 1' (lanes 1-4) or 'Hypertag Primer 2' in the presence of a BamHI Adapter. (B) Represents the results obtained using random hypertags.

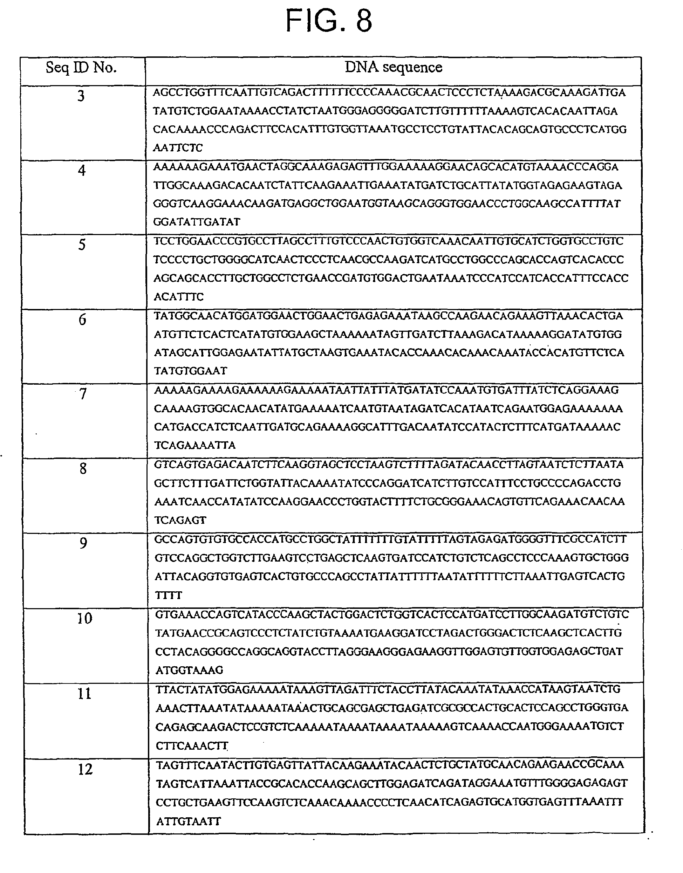

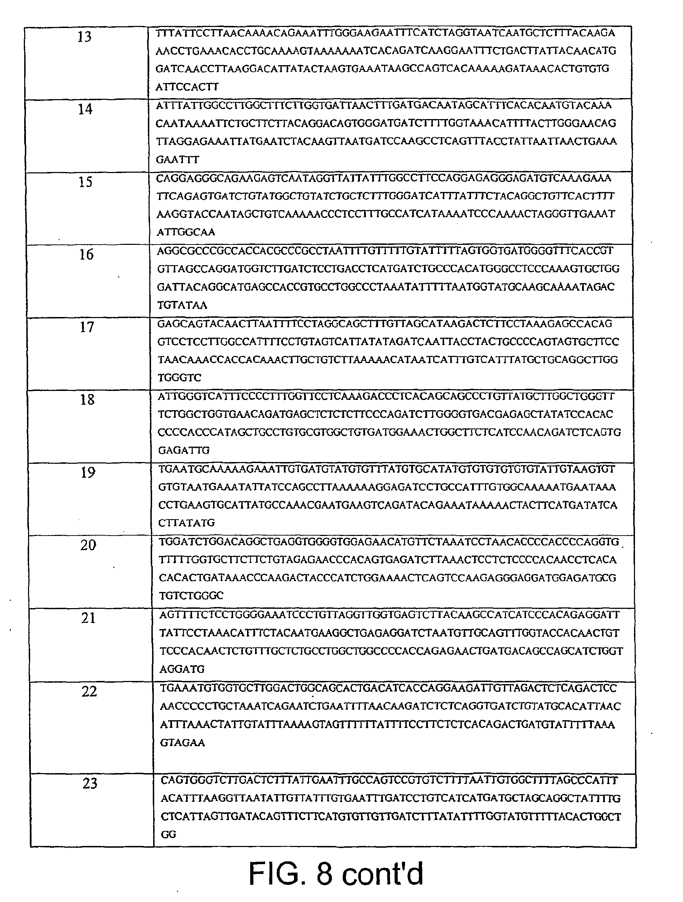

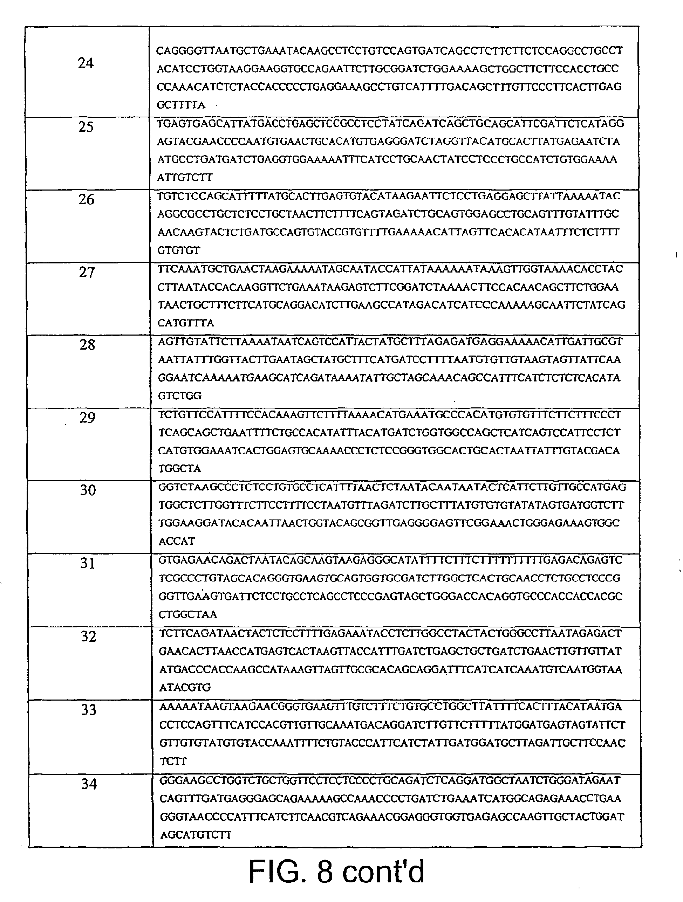

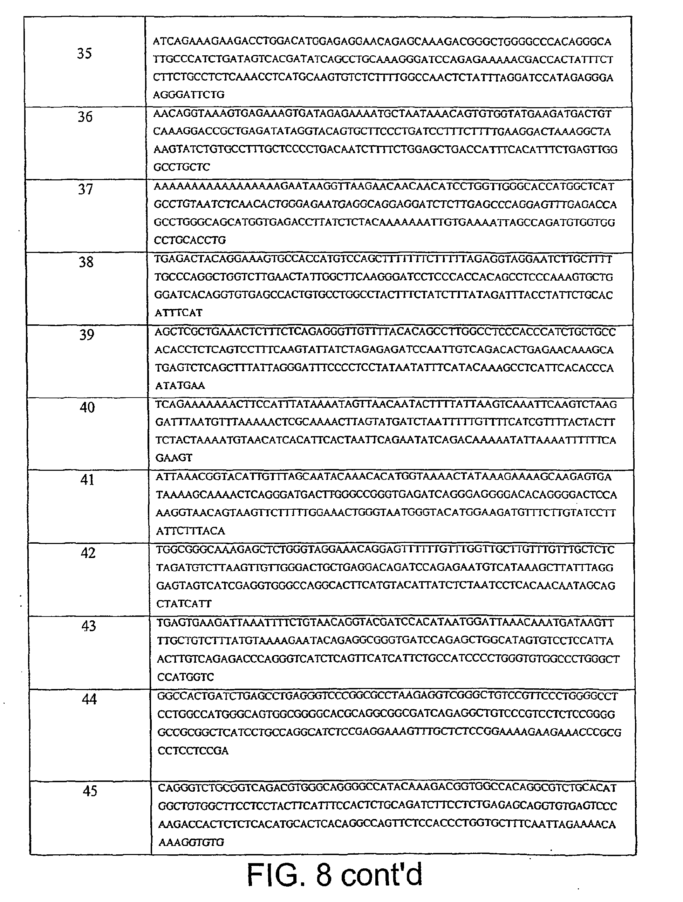

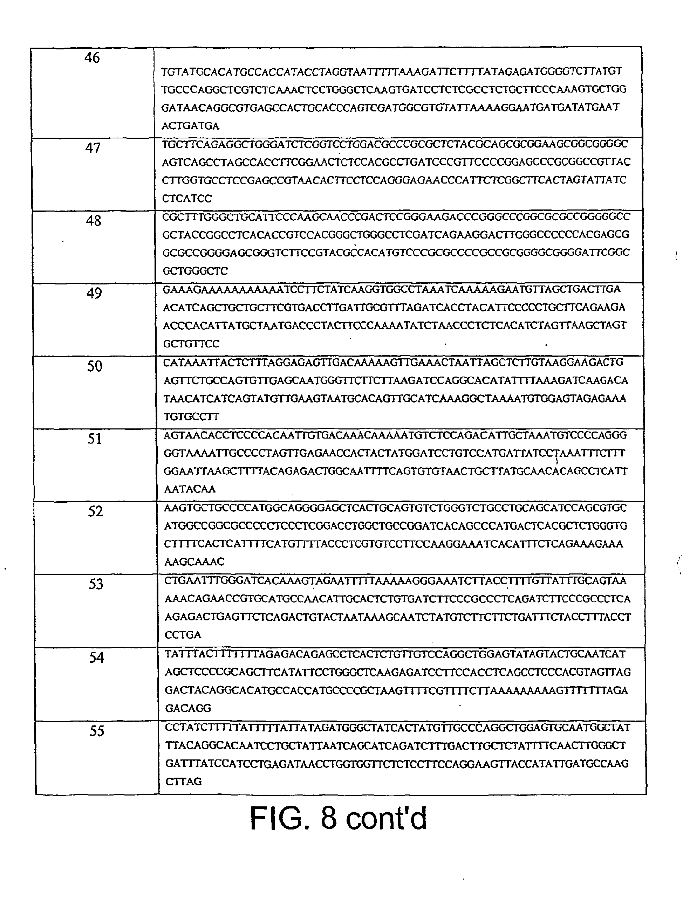

Figure 8, which represents a table, illustrates the sequences of 53 HS sequences (SEQ ID No. 3 to SEQ ID No. 55) identified by the methods of the present invention.

### EXAMPLES

### Example 1

### Large Scale Mapping of Genomic Regulatory Motifs in HeLa Cells Using a Hypersensitive Site Cloning Strategy

Until now the mapping of HSs using the 'indirect end-labeling' method (Wu, 1980) has exclusively focused on a small number of genes and no genome-global approaches for efficiently mapping HSs have been described. Global HS mapping data will allow unique insights concerning their location relative to transcription units, their sequence features and DNA structure and how the presence of HSs correlates with transcriptional state of adjacent genes. Such data is currently not obtainable on a large scale using the low-throughput methods that have been developed for studying HS patterns of individual genes. Implementation of a method that is capable of mapping of HSs in a comprehensive and genome-global manner is described. The principle is summarized in Figure 1. To create a genomic library highly enriched in DNA fragments derived from HSs, DNA present in nuclei is digested with a restriction enzyme under controlled conditions. To maximize cleavage in the majority of sites the restriction enzyme chosen will usually cut a 4 bp target sequence and produce a 'sticky end' suitable for cloning. One example of an enzyme fulfilling these criteria is Mbol. The Mbol cleaved genomic DNA is ligated to a BamHI-cleaved and phosphatased plasmid vector. This step covalently joins the DNA sequences adjacent to the Mbol cleavage site to the plasmid DNA, but does not yet result in the formation of a functional recombinant DNA molecule; the other end of the genomic fragment will usually be tens of kilobases away, may also be randomly sheared during the genomic DNA extraction step and will thus not be suitable for specifically joining the other BamHI site in the linearised plasmid. To create genomic fragments suitable for cloning the ligation mixture is cut to completion with EcoRI. This enzyme cuts at a defined site within the polylinker of the plasmid vector and also cuts every target site present in the genomic DNA. Since the DNA fragments adjacent to the Mbo I sequence are already ligated to the plasmid vector (see above), this step creates the condition for specifically joining the other end of the construct through intramolecular ligation. Due to the random orientation of the plasmid vector relative to the Mbol fragment during the first ligation approximately 50% of the clones are lost at this stage, but the other 50% of ligation products will contain specifically cloned Mbol-EcoRI genomic fragments. Transfection of the ligation products results in the creation of a library of genomic DNA fragments derived from a large variety of HSs. Determination of the insert sequence adjacent to the BamHl-Mbol junction of each clone establishes, after a search against human genome databases, the precise genomic location of the Mbol site and thus allows the positioning of a specific HS surrounding it.

### Materials and Methods

### Cell Culture, Nuclei Purification and HS Cleavage

HeLa S3 cells (obtained from the European Collection of Cell Cultures; ECACC Ref. No. 87110901) are grown to 80% confluency in 150 cm² flasks at 37°C in Dulbecco's Minimal Essential Medium/10% newborn calf serum (Sigma) in a 5% CO₂ humidified atmosphere. Before carrying out the procedure the appearance of cells is visually checked and their overall viability (>97%) assessed by trypan blue staining. After removing the medium the adherent cells are rinsed in Dulbecco's PBS (-Ca²⁺/Mg²⁺) and around 75% of the cells are detached by trypsin treatment. Isolation of nuclei is carried out using established protocols (Protocol 10.2; Carey and Smale, 1999). Briefly, 1.5 x 10⁷ cells are gently resuspended in 'NP40 lysis buffer' (10 mM Tris-HCl [pH 7.5]; 10 mM NaCl; 3 mM MgCl₂; 0.5% NP-40; 0.15 mM spermine-tetrachloride; 0.15 mM spermidine-trichloride) and incubated on ice for 10 minutes. The nuclei are purified from the lysate by low speed centrifugation and washed once in 'Restriction enzyme buffer' (50 mM Tris-HCl [pH 8.0]; 100 mM NaCl; 3 mM MgCl₂; 0.15 mM spermine-tetrachloride; 0.15 mM spermidine-trichloride). The purified nuclei are resuspended in 500 µl NEB Buffer 3 (100 mM NaCl, 50 mM Tris-HCl [pH7.9], 10 mM MgCl₂, 1 mM dithiothreitol) to yield a final volume of around 800 µl. Six 100 µl aliquots of the nuclei suspension are distributed into six separate microcentrifuge tubes which are subjected to the following treatments:

| | |
|---|---|
| Reaction 1: | no enzyme (negative control) |
| Reaction 2: | 100 units Mbo I (recombinant; 5 units/µl; New England Biolabs) |
| Reaction 3: | 50 units Mbo I |
| Reaction 4: | 25 units Mbo I |
| Reaction 5: | 10 units Mbo I |
| Reaction 6: | 5 units Mbo I |

Fragmentation of nuclease hypersensitive sites is initiated by incubating the reactions at 37°C for 10 minutes. Mbol is suitable for such studies because it specifically recognizes a 4 bp target site (5' GATC 3') and produces a single-stranded protruding end that can be ligated directly to BamHI-cleaved cloning vectors (see below). Previous work has established that proteins up to 500 kDa can diffuse freely through the nucleus (Seksek *et al*., 1997), suggesting that Mbo I (which is around 30 kDa in size) can in principle access hypersensitive sites throughout the genome during the duration of this incubation step. The cleavage reaction is terminated by the addition of EDTA (10 mM final concentration), proteinase K (750 µg), and SDS (2% final concentration). After an overnight incubation at 37°C genomic DNA is extracted with phenol/chloroform, treated with RNAase A, precipitated with ethanol and finally resuspended in TE (10 mM Tris-HCl [pH 8.0]; 1 mM EDTA).

### HS Library Construction

pLitmus38 (New England Biolabs) is digested to completion with BamHI and treated with alkaline phosphatase (Roche) to prevent self-ligation. 50 ng of the cut plasmid vector is ligated to 1 µg of HeLa S3 genomic DNA extracted from Mbol treated nuclei (from 'Reaction 3'; see above) for 1 hour at 16°C. After heat treatment at 65°C for 20 minutes the ligation products are digested to completion with EcoRI and extracted with phenol. The cleaved products are re-ligated overnight at 16°C at low concentration (final reaction volume 100 µl) to favour intramolecular ligation. 10 µl aliquots of the reaction are separately transformed into *E. coli* DH5α (transformation efficiency >10⁶ colonies per µg; 'Library Quality'; Gibco/BRL) to generate the 'HeLa S3 HS-Mbol' library.

### Characterization of HS Clones

Individual colonies from the HeLa S3 HS-Mbol library are randomly picked, streaked out to single colonies and grown up in small-scale (2 ml) liquid cultures. The plasmids are extracted and purified using the Qiaprep system (Qiagen). 1% of the recovered DNA is analyzed on a 0.7% agarose gel to eliminate plasmids that contain no detectable insert. The inserts of the remaining plasmids are characterized by automatic sequencing (MWG-Biotech) using the '-49 reverse' sequencing primer that allows the identification of the sequences adjacent to the BamHI/Mbo I cloning junction.

### Bioinformatics Analysis of HS Clones

The sequences obtained from individual HS clones are submitted for BLAST searches (Altschul *et al*., 1997) against the *Homo sapiens* genomic contig sequences (International Human Genome Sequencing Consortium, 2001) maintained by the NCBI ('Build 22'; http://wwwncbi.nlm.nih.gov). 10 kb of the chromosomal environment of the BLAST 'hits' with 99-100% are analyzed for the presence of previously identified transcription units by selecting the 'EST', 'GenomeScan' and 'GeneSeq' features of the web site. A region of 200 bp around the Mbo I site corresponding to the HS-cleavage site is further analyzed using a variety of web-based bioinformatics packages, including searches for the presence of transcription factor binding sites:
http://molsun1.cbrc.aist.go.jp/research/db/TFSEARCH.html
S/MARs (Scaffold/Matrix attachment regions):
   http://www.futuresoft.org/MAR-Wiz/
   and overall DNA curvature (Liu and Beveridge, 2001):
   http://ludwig.chem.wesleyan.edu/dna/

### Results

### Global Mapping of Chromatin Configurations

One of the most critical steps that determines to a large extent the success of the subsequent methods is the initial HS cleavage. This step has to be carried out under limiting conditions to avoid cleavage of target sequences outside HSs. This has been achieved by incubating isolated nuclei with a range of different restriction nuclease concentrations. Analysis of genomic DNA exposed to various amounts of Mbol shows that there is little, if any, difference in their appearances when analyzed on a 0.7% agarose gel, suggesting that the vast majority of DNA fragments are larger than 50 kb (Figure 2A). Thus, even at the highest nuclease concentrations used no extensive cleavage of the genomic DNA occurs. To monitor the extent of specific Mbol cleavage more quantitatively, a ³²P-labelled adapter containing a BamHl 'sticky' end is ligated to the samples. Under these conditions, DNA isolated from nuclei that are not exposed to Mbol fail to incorporate any of the labeled adapter molecules, whereas the amount of adapter incorporated into the genomic DNA correlates directly with the amount of Mbol used in the different nuclei fragmentation procedures (Figure 2B). Significantly, more than 98% of the radiolabelled adapter ligates to DNA fragments migrating at the size exclusion limit of the gel. This proves that the Mbol treatment results in the selective introduction of cuts separated by large distances, rather than in extensive cutting of small DNA sub-populations that may have become exposed in nuclei that may have lysed during the purification procedure. The interpretation of the gel result is also supported by the observation that ligation of the Mbol digested genomic DNA samples into BamHI cleaved plasmids fail to yield any recombinants. The Mbol digest of nuclei therefore does not lead by itself to the production of clonable fragments (as would be expected from unpackaged DNA since the 4 bp Mbol target sequence is expected to occur on average every 200 - 300 nucleotides, depending on local base composition).

### Chromosomal and Genomic Distribution of Mapped HSs

The sequences obtained from 67 clones derived from a HeLa S3 HS-library were checked against the publicly available human genome sequence to locate their genomic origin. The chromosomal locations of 49 clones could be assigned to unique chromosomal locations, whereas the remaining 18 clones either had no match in the human genome database, corresponded to unmapped contigs or could not be reliably assigned due to ambiguities in the genome database. As expected, the HS candidates are evenly distributed. As expected, the HS candidates are evenly distributed over the majority of the chromosomes. Some chromosomes have not had HSs mapped to yet, but this is almost certainly due to the relatively small number of clones characterized rather than due to systematic experimental bias.

### Intrinsic Sequence Features of HSs and Relationship to Surrounding Transcription Units

The location of experimentally mapped and computer predicted transcription units within 10 kb surrounding the HS is analyzed using the 'EST', 'GenomeScan' and 'GeneSeq' display features of the NCBI genome server. Previously characterized HSs in different genes have been mapped either towards the 5' or 3' regions of genes, or even within intronic regions (Picard and Schaffner, 1984). In this study all the characterized clones mapped to defined genomic locations that display the expected characteristics of mapping close to or within introns of experimentally-defined of computer-predicted transcription units. A selection of five randomly chosen examples is shown in Figure 3. The positions of the mapped sites are compatible with their potential role as transcriptional regulatory motifs, such as enhancers and insulators. Many transcriptional control sequences also coincide with nuclear matrix attachment regions (S/MARs; Gasser and Laemmli, 1986; Li et al., 1999). Computer-aided analysis of 100 nucleotide sequence stretches surrounding the HS-Mbol site of 26 clones revealed numerous sequence motifs characteristic of S/MARs in several clones. Several clones are found to contain sequences compatible with replication consensus motifs, curved or kinked DNA sequences (Figure 4).

The data obtained from global studies provides a significant amount of added value, especially if tissue- and stage specific HS information is directly linked to specific genomic regions. This annotation scheme could be even further increased in value by the addition of cross-species epigenomic data (e.g. based on the systematic mapping of HSs in mouse and other vertebrate genomes) where it will be possible to look at a range of different tissues and endogenous cell types much more readily. Integration of cell type-specific chromatin state information with information obtained from other global analysis technologies, such as gene expression patterns based on DNA microarray and proteome projects, will reveal new insights into the mechanisms of gene expression in eukaryotic organisms.

Thus, it is demonstrated that a hypersensitive site cloning strategy can be used for the large-scale mapping of genomic regulatory motifs.

### Example 2

### Identification of Hypersensitive Sites with 'Hypertag Display'

HSs are useful experimental markers for the presence of functional regulatory regions in eukaryotic genomes. HSs have been mapped, often in great detail, for a number of genes, but the labour-intense nature of the procedure has up to now precluded a genome-wide approach. The method described in Example 1 for constructing and characterizing libraries containing DNA selectively cloned from HSs promises a solution to this problem. This method allows the establishment of a comprehensive map of genomic HS locations in a particular cell type. Since many genes involved in regulatory events are selectively expressed in a tissue- and cell type-specific manner it is important to develop additional methods for comparing HSs patterns in a large number of different cells. This concept is foundation of the 'hypertag display' method described here.

The principle of hypertag display is shown schematically in Figure 5; DNA present in HSs is selectively cut with the restriction enzyme Mbo I, recognizing the 4 bp target sequence ^{5'}GATC^{3'}. Ligation of a compatible BamHI adapter molecule to the cleaved ends results in the selective tagging of each cleaved Mbo I site with a fragment of predetermined and known sequence. The tagged fragment is subsequently amplified by PCR using an oligonucleotide complementary to the adapter molecule and a second oligonucleotide (the 'Hypertag' primer) that is complementary to a sequence located next to a previously mapped HS. The required local sequence information can be derived from data obtained through the HS library approach (Example 1). This procedure results in the production of a PCR amplification product of defined size (a 'hypertag'). Note, however, that the synthesis of the PCR product will depend entirely on a successful Mbo I cleavage event, which in turn is dependent on the local chromatin environment. If cleavage occurs, because the target sequence is in HS-configuration, a specific PCR fragment will result. If the Mbo I target site is inaccessible due to dense chromatin packaging and absence of suitable HSs, no PCR product will be detected. The production of an amplified PCR fragment thus serves as a specific and sensitive beacon for the presence/absence of a predetermined HS within an eukaryotic genome.

The method may be used to compare the state of a particular HS in various cell types (Figure 5), but can also be adapted to the detection of several different HSs within a sample by designing the hypertag primers to yield amplification products of different sizes (Figure 6). Finally, by choosing a short hypertag oligonucleotide (e.g. 6-8 nucleotides long) to reduce the priming specificity it is also possible to display a range of 'random' (i.e. yet uncharacterized) HSs for comparative assays.

### Materials and Methods

### Cell Culture, Nuclei Purification and HS Cleavage

### The hypersensitive-site cleaved DNA samples were prepared as described in Example 1.

### Preparation of DNA Samples for Hypertag Display

There are at least two requirements for oligonucleotide adapters, including: i) presence of a single-stranded cohesive end that will base pair specifically with the DNA fragment ends produced by the genomic restriction nuclease fragmentation reaction, and ii) they need to have a unique sequence that is different from any other sequence found in eukaryotic genomes and can therefore act as a specific primer binding site during the PCR reaction. With these criteria in mind, a BamHI adapter is designed as follows:
5'OH *CGCCAGGGTTTTCCCAGTCACGAC* 3'OH

The residue underlined corresponds to the 5'OH group of the top strand oligonucleotide that can be labeled with, for example, radioactive and/or fluorescent labels to facilitate the subsequent detection of the PCR products.
3'OH *GCGGTCCCAAAAGGGTCAGTGCTGCTA*G 5'OH

The residue underlined may be phosphorylated to ensure the formation of a covalent link between the adapter oligonucleotide and the cleaved genomic DNA during the ligation reaction.

The sequences shown in italics are derived from bacteriophage M13 and used extensively as a 'universal' sequencing primer in standard plasmids and bacteriophage cloning vectors. The motif does not display any obvious homologies to any sequenced eukaryotic genome and will therefore not cross-hybridize to endogenous loci during PCR reactions. Any other unique oligonucleotide sequence not occurring in the tested genome is suitable for this task. 8 pmoles of the 'bottom strand' oligonucleotide of the BamHI Adapter (containing the ^{5'}GATC^{3'} single-stranded extension) are phosphorylated in a final volume of 10 µl in a microcentrifuge tube at 37°C for 10 minutes in the presence of 2 µl of [γ-³²P] rATP (New England Nuclear; 3000 Ci/mmol at 10 mCi/ml) and 10 units of T4 polynucleotide kinase (Promega). The reaction is stopped by the addition of 1 µl of 0.5 M EDTA.Na₂ and 89 µl of TE buffer. The phosphorylated oligonucleotide is purified away from unincorporated rATP using a G25 microspin column (Amersham-Pharmacia). 8 pmoles of the 'top strand' oligonucleotide are added to the column eluate and, after heating to 75°C for 5 minutes, the mixture is allowed to cool to room temperature to anneal the two strands. 2 µl of the annealed BamHI adapter is ligated to 1 µg of hypersensitive site Mbol-cleaved genomic DNA at 16°C for 1 hour in a final volume of 10 µl (note that due to the small number of cleaved Mbo I sites in the genomic DNA the adapter is likely to be in substantial excess and therefore no alkaline phosphatase treatment of the genomic DNA is required to prevent ligation of genomic fragments to each other). 1 µl of the ligation reaction containing the adapter-tagged DNA is used for each PCR reaction.

### PCR amplification

PCR reactions are carried out in a total reaction volume of 50 µl with a Stratagene Robocycler using the MβP 'EasyStart' system (obtained from Merck). Amplification is carried out for 40 cycles (45 seconds at 95°C; 45 seconds at 55°C; 1 minute at 72°C). 10 µl of each PCR reaction are analyzed on a 0.7% agarose/TBE gel.

### Results

The experimental implementation of the scheme is carried out using two hypertag primers designed to detect different HSs that were previously detected in HeLa cell DNA using the high-throughput characterization strategy of an HS library (Example 1). The hypertag primers, in combination with the adapter-specific oligonucleotide, gives rise to defined hypertags when DNA prepared from Mbol-treated nuclei is used. On the other hand, the same primer combination fails to reveal any discrete amplification products when DNA from untreated nuclei is added to the reaction (Figure 7A). The different sizes of the two hypertags paves the way for analyzing a multitude of hypertags originating from a variety of HSs in a single electrophoresis lane.

The limited number of Mbol cuts present in the HS-cleaved DNA poses a substantial challenge for their detection and usually requires 40 rounds of amplification for analysis. The sensitivity and signal/noise ratio of the reactions may be improved by optimising the amounts of BamHI adapter added to the genomic DNA samples, by carrying out the PCR reactions under different conditions (such as changes in annealing temperature, use of different DNA polymerases, effects of different additives etc.). By carrying out the PCR reaction in the presence of labeled dNTPs the sensitivity of the assay may be increased further.

Figure 7B shows results obtained by carrying out the PCR reactions with an adapter-specific oligonucleotide in the presence of three different short oligonucleotide primers with the aim of displaying multiple hypertags of 'random' (i.e. not pre-selected) origin. PCR reactions that either do not contain the adapter-specific primer, or genomic DNA from untreated nuclei fail to display a detectable banding pattern (data not shown). These negative controls support the interpretation that the 'random' hypertag patterns are derived from HS-specific genomic locations. Cloning and sequencing of several of the fragments generated in this procedure is currently in progress to obtain more information regarding their genomic origin. The complexity of the profile pattern can be increased by variations in the PCR conditions, changes in the design of the short oligonucleotides, or by increasing the number of oligonucleotides used in the assay.

The 'hypertag display' method is a fast and powerful method of checking either the status of a set of predetermined HSs or for creating a specific profile of yet uncharacterized HSs. When combined with the information about HS map locations obtained from HS-libraries (Example 1) the hypertag display method creates a powerful platform technology that is capable of creating a specific HS 'fingerprint' from any eukaryotic genome. The method is highly amenable to automation and can thus be applied in high-throughput drug screening operations. A collection of HS-specific hypertag primers can be used to probe the state of a plurality of the most variable and diagnostically-useful HSs in a single round of experiments.

Thus, it is demonstrated that hypersensitive sites may be identified with 'Hypertag Display'.

### Example 3

### Identification of Hypersensitive Sites with RLGS

### Cell Culture and HS Cleavage

HeLa S3 cells (obtained from the European Collection of Cell Cultures; ECACC Ref. No. 87110901) are grown to 80% confluency in 150 cm² flasks at 37°C in Dulbecco's Minimal Essential Medium/10% newborn calf serum (Sigma) in a 5% CO₂ humidified atmosphere. Before carrying out the procedure the appearance of cells is visually checked and their overall viability (>97%) assessed by trypan blue staining. After removing the medium the adherent cells are rinsed in Dulbecco's PBS (-Ca²⁺/Mg²⁺) and around 75% of the cells are detached by trypsin treatment.

### Cell Permeablisation and Fragmentation

Cells are temporarily permeabilised by lysolecithin treatment.

Aliquots of the permeabilised cells are distributed into six separate microcentrifuge tubes which are subjected to the following treatments:

| | |
|---|---|
| Reaction 1: | no enzyme (negative control) |
| Reaction 2: | 100 units Mbo I (recombinant; 5 units/µl; New England Biolabs) |
| Reaction 3: | 50 units Mbo I |
| Reaction 4: | 25 units Mbo I |
| Reaction 5: | 10 units Mbo I |
| Reaction 6: | 5 units Mbo I |

The endonuclease cleaves DNA target sites within the cells that are exposed in HSs.

After 30-60 minutes the cells are permeabilised again and incubated with DNA polymerase (Klenow fragment) and a radioactively labelled dNTP. This step leads to the selective incorporation of the radioisotope into the genomic DNA at the sites that have been cleaved by the restriction enzyme.

### DNA Extraction

After 30-60 minutes the cleavage reaction is terminated by the addition of EDTA (10 mM final concentration), proteinase K (750 µg), and SDS (2% final concentration) and the cells are lysed. Genomic DNA is extracted with phenol/chloroform, treated with RNAase A, precipitated with ethanol and finally resuspended in TE (10 mM Tris-HCl [pH 8.0]; 1 mM EDTA).

### DNA Cleavage

The DNA is cleaved with a second restriction enzyme with a 6 bp recognition sequence to reduce the average size of the fragments.

### RLGS

The fragment mixture is subjected to RLGS in accordance with Hatada et al. (1991) and includes an in-gel digest with a 4 bp cutting restriction enzyme to produce a two-dimensional gel image.

### Analysis

The radiolabelled fragments are subsequently analysed by methods - such as phospho-imaging and/or autoradiography.

### Results

Each detectable spot on the two-dimensional gel image corresponds to a cleaved HS, and therefore the entire gel image represents a global map of the chromatin topology present in the original cell sample.

### Example 4

Using the methods of the present invention, 53 HSs are identified comprising the sequences set forth in SEQ ID No. 3 to SEQ ID No. 55.

### DISCUSSION

Despite the obvious fundamental biological importance of HSs, the general field is not particularly densely populated with investigators. This is partially due to historic reasons. A small number of laboratories have been working on HSs for a considerable amount of time and this has resulted in a situation where the bulk of the published work is restricted to a small number of genes. In addition, the main thrust of the laboratories studying them has been towards obtaining higher resolution 'snapshots' of the molecular micro anatomy of individual HSs.

The strategy proposed here is diametrically opposed to this aim and thus opens up a vast and previously unexplored research area. The present invention relates to the analysis of a large set (thousands and tens of thousands) of HSs present in particular cell types in order to study the HS patterns expressed in different tissues and cell types at various developmental stages. Here, it is to be noted that approximately 1 % of the genomic DNA is estimated to be present in HS conformations (Gross and Garrard, 1988). A major innovative feature of one aspect of the present invention - which may be termed 'Global Analysis of Chromatin Topology' ('GACT') or 'Hypergenomic Display' ('HD') - is that it allows an efficient and genome-wide survey of HSs. The large number of data points derived from a single experiment will make such a procedure ideally suited for bioinformatic processing and analysis. Furthermore, the method can be fully automated using purpose-built robotic systems, which will allow it to be applied for high-throughput drug screening strategies.

As indicated above, HSs are the main regulatory entry points into the genome the availability of such a method will have a considerable impact on many areas of medicine and biotechnology. Like many other molecular biology techniques the proposed methods will be universally applicable to address numerous key questions in any eukaryotic organism, ranging from yeast to humans.

The medical and biotechnological applications for GACT are tremendous. As mentioned above, HSs are the main regulatory access points for external agents to the genome and so the availability of GACT will have a considerable impact on many areas of medicine and biotechnology. Like many other molecular biology techniques it will be universally applicable to address numerous questions in any eukaryotic organism, ranging from yeast to humans.

The examples given below represent a brief overview of potential applications:
- Epigenomic profiling of cancer types to optimise therapeutic treatments.
- Exploration of epigenetic changes leading to carcinogenesis.
- Development of drugs that influence chromatin topology.
- Stepwise preparation of genomes for controlled differentiation of embryonic stem cells along predetermined pathways and of controlled dedifferentiation of somatic cells to increase their pluripotency (e.g. somatic stem cell therapy).
- Controlled redifferentiation of cell types to different fates through creating suitable epigenomic states.
- Studying epigenetic effects of ageing.
- Choosing DNA fragments for transgenic animals and for human gene therapy that contain the appropriate control signals and chromatin boundary elements for correct expression of gene in recipient organism.

The experimental approaches described above will constitute the main area of application of the new technology. There are, however, additional topics that could be addressed. By way of example, one additional topic that could be addressed relates to topoisomerases. Topoisomerases (such as topoisomerase I and II) are enzymes that are capable of rearranging chromatin topology and are usually expressed in proliferating cells. The enzymes cleave their DNA substrate specifically at a 16 bp consensus sequence and then allow free rotation of the cleaved ends relative to each other which leads to a decrease in the superhelical density. Afterwards the two cleaved DNA ends are covalently linked to each other to restore a continuous DNA molecule. Topoisomerases can be induced to carry out the cleavage reaction under *in vivo* conditions or in purified nuclei in the presence of sodium dodecyl sulphate. It is therefore possible to apply the methods described to analyse the topoisomerase induced cleavage patterns on a global scale, which would reveal the genomic sites to which topoisomerase where originally bound. Such data can be very useful for complementing the global HS mapping approach by providing additional information concerning the epigenomic regulation of the genome in particular cell types.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### REFERENCES

Altschul, S.F., Madden, T.L., Schaffer, A.A., Zhang, J., Zhang, Z., Miller, W., and Lipman, D.J. (1997). Gapped BLAST and PSI-BLAST: a new generation of protein database search programs. Nucl. Acids Res. 25, 3389-3402.
Baltimore, D. (2001). Our genome unveiled. Nature 409, 814-816.
Bonifer, C. (2000). Developmental regulation of eukaryotic gene loci. Trends Genet. 16, 310-315.
Boyes, J., and Felsenfeld, G. (1996). Tissue-specific factors additively increase the probability of the all-or-none formation of a hypersensitive site. EMBO J. 15, 2496-2507.
Carey, M., and Smale, S.T. (1999). Transcriptional Regulation in Eukaryotes: Concepts, Strategies and Techniques. Cold Spring Harbor Laboratory Press. Cold Spring Harbor, New York.
Dhar, V., Nandi, A., Schildkraut, C.L., and Skoultchi, A.I. (1990). Erythroid-specific nuclease hypersensitive sites flanking the human β-globin domain. Mol. Cell. Biol. 10, 4324-4333.
Filipski, J., Leblanc, J., Youdale, T., Sikorska, M., and Walker, P.R. (1990). Periodicity of DNA folding in higher order chromatin structures. EMBO J. 9, 1319-1327.
Gasser, S.M., and Laemmli, U.K. (1986). Cohabitation of scaffold binding regions with upstream/enhancer elements of three developmentally regulated genes of D. melanogaster. Cell 46, 521-530
Gross, D.S., and Garrard, W.T. (1988). Nuclease hypersensitive sites in chromatin. Ann. Rev. Biochem. 57,159-197.
Hatada, I., Hayashizaki, Y., Hirotsune, S., Komatsubara, H., and Mukai, T. (1991). A genomic scanning method for higher organisms using restriction sites as landmarks. Proc. Natl. Acad. Sci. USA 88, 9523-9527.
Huber, M.C., Graf, T., Sippel, A.E., and Bonifer, C. (1995). Dynamic changes in the chromatin of the chicken lysozyme gene during differentiation of multipotent progenitors to macrophages. DNA Cell Biol. 14, 397-402.
International Human Genome Sequencing Consortium (2001). Initial sequencing and analysis of the human genome. Nature 409, 860-921.
Kontaraki, J., Chen, H.-H., Riggs, A., and Bonifer, C. (2000). Chromatin fine structure profiles for a developmentally regulated gene: reorganization of the lysozyme locus before transactivator binding and gene expression. Genes Dev. 14, 1206-2122.
Liang, P., and Pardee, A.B. (1992). Differential display of eukaryotic messenger RNA by means of the polymerase chain reaction. Science 257, 967-971.
Liu, Y., and Beveridge, D.L. (2001). A refined prediction model for gel retardation of DNA oligonucleotides from dinucleotide step parameters: reconciliation of DNA bending models with crystal structure data. J. Biomol. Struct. Dyn. 18, 505-526.
Ma, D., Xing, Z., Liu, B., Pedigo, N.G., Zimmer, S.G., Bai, Z., Postel, E.H., and Kaetzel, D.M. (2002). NM23-H1 and NM23-H2 repress transcriptional activities of nuclease-hypersensitive elements in the platelet-derived growth factor-A promoter. J. Biol. Chem. 277, 1560-1567.
Mielke, C., Maass, K., Tümmler, M., and Bode, J. (1996). Anatomy of highly expressing chromosomal sites targeted by retroviral vectors. Biochemistry 35, 2239-2252.
Phylactides, M., Rowntree, R., Nuthall, H., Ussery, D., Wheeler, A., and Harris, A. (2002). Evaluation of potential regulatory elements identified as DNase I hypersensitive sites in the CFTR gene. Eur. J. Biochem. 269, 553-559.
Picard, D., and Schaffner, W. (1984). A lymphocyte-specific enhancer in the mouse immunoglobulin kappa gene. Nature 307, 80-82.
Schubeler, D., Lorincz, M.C., Cimbora, D.M., Telling, A., Feng, Y.-Q., Bouhassira E.E., and Groudine, M. (2000). Genomic targeting of methylated DNA: influence of methylation on transcription, replication, chromatin structure, and histone acetylation. Mol. Cell. Biol. 20, 9103-9112.
Seksec, O., Biwersi, J., and Verkman, A.S. (1997). Translational diffusion of macromoleculesized solutes in cytoplasm and nucleus. J. Cell Biol. 138, 131-142.
Simonsson, T., Pecinka, P. and Kubista, M. (1998). DNA tetraplex formation in the control region of c-myc. Nucl. Acids Res. 26, 1167-1172.
Smith, D.J., Nuthall, H.N., Majetti, M.E., and Harris, A. (2000). Multiple potential intragenic regulatory elements in the CFTR gene. Genomics 64, 90-96.
Stalder, J., Larsen, A., Engel, J.D., Dolan, M., Groudine, M., and Weintraub, H. (1980). Tissue-specific cleavage in the globin chromatin domain introduced by DNAase I. Cell 20, 451-460.
Svetlova, E., Avril-Fournout, N., Ira, G., Deschavanne, P., and Filipski, J. (1998). DNase-hypersensitive sites in yeast artificial chromosomes containing human DNA. Mol. Gen. Genet. 257, 292-298.
Wolffe, A.P., and Hayes, J.J. (1999). Chromatin disruption and modification. Nud. Acids Res. 27, 711-720.
Wu, C. (1980). The 5' ends of Drosophila heat shock genes in chromatin are hypersensitive to DNAase I. Nature 286, 854-860.
Zhang, L., Spratt, S.K., Liu, Q., Johnstone, B., Qi, H., Raschke, E.E., Jamieson, A.C., Rebar, E.J., Wolffe, A.P., and Case, C.C. (2000). Synthetic zinc finger transcription factor action at an endogenous chromosomal site. J. Biol. Chem. 275, 33850-33860.

The invention is further described in the following numbered paragraphs.
1. A method of determining chromatin structure of a nucleotide sequence comprising the steps of (i) fragmenting the nucleotide sequence at multiple HSs; and (ii) analysing fragments formed in step (i) from a plurality of sequences.
2. A method according to paragraph 1 wherein the nucleic acid is fragmented by exposing the sample to at least one fragmenting means capable of fragmenting the nucleic acid at multiple HSs.
3. A method according to paragraph 2 wherein the fragmenting means is an enzyme.
4. A method according to paragraph 3 wherein the fragmenting means is a restriction enzyme.
5. A method according to any one of the preceding paragraphs wherein in step (i) the sample is treated with a plurality of sequence specific restriction enzymes with different target site specificities capable of fragmenting the nucleic acid at multiple HSs.
6. A method according to any one of the preceding paragraphs wherein the fragments are analysed by incorporating detectable nucleotides into the fragments at the HSs and then detecting the incorporated nucleotides.
7. A method of determining chromatin structure in a nucleic acid sample comprising the steps of (i) treating the sample with a first restriction enzyme capable of fragmenting the nucleic acid at multiple HSs; (ii) incorporating detectable nucleotides into the fragments formed in step (i); (iii) isolating the nucleic acid from the sample ; (iv) treating the nucleic acid from step (iii) with a second restriction enzyme; and (v) analysing the fragments from step (iv) from a plurality of genes.
8. A method of determining chromatin structure in a nucleic acid sample comprising the steps of (i) treating the sample with a first restriction enzyme capable of fragmenting the nucleic acid at multiple HSs; (ii) incorporating detectable nucleotides into the fragments formed in step (i); (iii) isolating the nucleic acid from the sample ; (iv) treating the nucleic acid from step (iii) with a second restriction enzyme; and (v) analysing the fragments from step (iv) from a plurality of genes by applying the fragmented nucleic acids from step (iv) to gel electrophoresis incorporating an in-gel digest.
9. A method of determining chromatin structure in a nucleic acid sample comprising the steps of (i) treating the sample with a first restriction enzyme capable of fragmenting the nucleic acid at multiple HSs; (ii) incorporating a target nucleotide sequence into the fragments formed in step (i); (iii) isolating the nucleic acid; (iv) selectively amplifying portions of the nucleic acid from step (iii) using a first primer capable of binding to the target nucleotide sequence and a second primer capable of binding to another portion of the nucleic acid; and (v) analysing the amplified portions from step (iv).
10. A method of determining a characteristic of a nucleic acid sample comprising the steps of (i) treating the nucleic acid sample to fragment the nucleic acid therein at multiple HSs; (ii) analysing fragments formed in step (i) from a plurality of genes; and (iii) using the pattern of fragments from step (ii) to determine the characteristic.
11. A method of diagnosing a disease in subject wherein the disease is associated with an altered chromatin structure the method comprising the steps of (i) treating a nucleic acid sample from the subject to fragment the nucleic acid therein at multiple HSs; (ii) analysing fragments formed in step (i) from a plurality of genes; and (iii) using the fragment pattern to diagnose the disease.
12. A method of monitoring the progress of a disease in a subject, the method comprising the steps of (i) treating a nucleic acid sample from the subject to fragment the nucleic acid therein at multiple HSs; (ii) analysing fragments formed in step (i) from a plurality of genes; and (iii) using the pattern of fragments from step (ii) to determine the stage of the disease.
13. A method of testing the efficacy of a putative therapeutic agent which may be suitable for treating a disease in a subject associated with an altered chromatin structure comprising using said agent in a method according to any one of the preceding paragraphs and then determining if said agent is capable of modulating said HS.
14. A method of identifying a chromatin modulating (e. g. modifying) agent capable of modulating (e. g. modifying) chromatin structure, the method comprising (i) treating a nucleic acid sample to fragment the nucleic acid therein at multiple HSs, wherein the sample is either pre-treated or not pre-treated with the chromatin modulating (e. g. modifying) agent; (ii) analysing the fragments formed in step (i); and (iii) comparing the fragments analysed in step (ii) to identify the chromatin modulating (e. g. modifying) agent.
15. A method of identifying chromatin modulating (e. g. modifying) agent binding sites, the method comprising determining the chromatin structure in a nucleic acid sample with and without treatment with a chromatin modulating (e. g. modifying) agent and comparing the resulting fragment patterns to identify the location of the binding sites in the original sample.
16. A chromatin modulating (e. g. modifying) agent identified using the method of any one of paragraphs 14 to 16.
17. A process comprising the steps of : (i) performing the method according to any one of paragraphs 14-16; (ii) identifying one or more agents capable of diagnosing and/or modulating said HS; and (iii) preparing a quantity of those one or more agent.
18. A process comprising the steps of: (i) performing the method according to any one of paragraphs 14-16; (ii) identifying one or more agents capable of diagnosing and/or modulating said HS; and (iii) preparing a pharmaceutical composition comprising those one or more identified agents.
19. A process comprising the steps of: (i) performing the method according to any one of paragraphs 14-16; (ii) identifying one or more agents capable of diagnosing and/or modulating said HS; (iii) modifying those one or more identified agents; and (iv) optionally preparing a pharmaceutical composition comprising those one or more modified agents.
20. A method of treating a disease associated with chromatin structure in a subject, the method comprising administering to the subject an effective amount of a chromatin modulating (e. g. modifying) agent capable of modulating (e. g. modifying) the chromatin structure to a non-diseased form.
21. A pharmaceutical composition comprising an agent according to paragraph 17 and a pharmaceutical acceptable carrier, diluent, excipient or adjuvant or any combination thereof.
22. A method of preventing and/or treating a disorder comprising administering an agent according to paragraphs 17 or a pharmaceutical according to paragraph 21 wherein said agent or said pharmaceutical is capable of modulating an HS to cause a beneficial preventative and/or therapeutic effect.
23. Use of an agent according to paragraph 17 in the preparation of a pharmaceutical composition for the treatment of an HS related disorder.
24. A method of preparing a HS library comprising the steps of (i) treating a nucleic acid sample to fragment the nucleic acid therein at multiple HSs; (ii) ligating the nucleic acid fragments formed in step (i) from a plurality of sequences in to a vector; and optionally (iii) transforming the vector into a host cell to provide a library of cells.
25. A library of HSs wherein each library entry comprises HSs from a plurality of genes.
26. A library of HSs according to paragraph 25 comprising one or more of the HS sequences set forth in SEQ ID No. 3 to SEQ ID No. 55.

## Claims

1. A method of determining chromatin structure of a nucleic acid comprising the steps of (i) fragmenting the nucleotide sequence at multiple hypersensitive sites; and (ii) visualizing, identifying, detecting, mapping and/or sequencing of fragments formed in step (i) from at least 3 genes.

2. A method according to claim 1 wherein the nucleic acid is fragmented by exposing the nucleic acid to at least one fragmenting means capable of fragmenting the nucleic acid at multiple hypersensitive sites.

3. A method according to claim 2 wherein the fragmenting means is an enzyme.

4. A method according to claim 3 wherein the fragmenting means is a restriction enzyme.

5. A method according to any one of the preceding claims wherein in step (i) the sample is treated with a plurality of sequence specific restriction enzymes with different target site specificities capable of fragmenting the nucleic acid at multiple hypersensitive sites.

6. A method of determining chromatin structure in a nucleic acid sample comprising the steps of (i) treating the sample with a first restriction enzyme capable of fragmenting the nucleic acid at multiple hypersensitive sites; (ii) incorporating a target nucleotide sequence into the fragments formed in step (i); (iii) isolating the nucleic acid; (iv) selectively amplifying portions of the nucleic acid from step (iii) using a first primer capable of binding to the target nucleotide sequence and a second primer capable of binding to another portion of the nucleic acid; and (v) analysing the amplified portions from step (iv) from at least 3 genes.

7. A method of determining a cellular characteristic that correlates with chromatin structure in a nucleic acid sample originating from a cell comprising the steps of (i) treating the nucleic acid sample to fragment the nucleic acid therein at multiple hypersensitive sites; (ii) analysing fragments formed in step (i) from at least 3 genes; and (iii) using the pattern of fragments from step (ii) to determine the characteristic.

8. A method of diagnosing a disease in a subject wherein the disease is associated with an altered chromatin structure the method comprising the steps of (i) treating a nucleic acid sample from the subject to fragment the nucleic acid therein at multiple hypersensitive sites; (ii) analysing fragments formed in step (i) from at least 3 genes; and (iii) using the fragment pattern to diagnose the disease.

9. A method of monitoring the progress of a disease associated with an altered chromatin structure in a subject, the method comprising the steps of (i) treating a nucleic acid sample from the subject to fragment the nucleic acid therein at multiple hypersensitive sites; (ii) analysing fragments formed in step (i) from at least 3 genes; and (iii) using the pattern of fragments from step (it) to determine the stage of the disease.

10. A method of testing the efficacy of a putative therapeutic agent which may be suitable for treating a disease associated with an altered chromatin structure in a subject comprising using said agent in a method according to any one of the preceding claims and then determining if said agent is capable of modifying said hypersensitive site.

11. A method of identifying a chromatin modulating (e.g. modifying) agent capable of modulating (e.g. modifying) chromatin structure, the method comprising (i) treating a nucleic acid sample to fragment the nucleic acid therein at multiple hypersensitive sites, wherein the sample is either pre-treated or not pre-treated with the chromatin modulating (e.g. modifying) agent; (ii) analyzing the fragments formed in step (i); and (iii) comparing the fragments analysed in step (ii) to identify the chromatin mediating (e.g. modifying) agent.

12. A method of preparing a hypersensitive site library comprising the steps of (i) treating a nucleic acid sample to fragment the nucleic acid therein at multiple hypersensitive sites; (ii) ligating the nucleic acid fragments formed in step (i) from at least 3 genes in to a vector; and optionally (iii) transforming the vector into a host cell to provide a library of cells.

13. A library of hypersensitive sites obtainable by the method according to claim 12 wherein each library entry comprises hypersensitive sites from a plurality of sequences.

14. A library of hypersensitive sites according to claim 13 comprising one or more of the hypersensitive site sequences set forth in SEQ ID No. 3 to SEQ ID No. 55.
